# EUROPEAN PATENT APPLICATION

(11) **EP 3 862 366 A1**
(43) Date of publication of application: **11.08.2021**
(21) Application number: 19870017.1
(22) Date of filing: 04.10.2019
(51) Int. Cl.: C07K 16/46, A61K 39/395, A61P 35/00, C07K 16/28, C07K 16/30, C07K 19/00, C12N 15/13, C12N 15/62, C12P 21/08

(54) **CANCER-STEM-CELL-SPECIFIC ANTIBODY**

(30) Priority: 05.10.2018 JP 2018189834
(71) Applicant: Sapporo Medical University, Sapporo-shi, Hokkaido 060-8556 (JP)
(72) Inventor: TSUKAHARA, Tomohide, Sapporo-shi, Hokkaido 060-8556 (JP); TORIGOE, Toshihiko, Sapporo-shi, Hokkaido 060-8556 (JP)
(74) Representative: Zech, Stefan Markus
(86) International application number: PCT/JP2019/039400
(87) International publication number: WO 2020/071554

(57) **Abstract**

The present invention pertains to: an antibody that specifically recognizes cancer stem cells; a pharmaceutical composition containing the antibody, in particular a pharmaceutical composition for treating cancer; the utilization of these; a method for treating cancer, the method targeting cancer stem cells; etc. The above problem was solved by providing an antibody that recognizes a complex of a cancer stem cell antigen peptide and an MHC, and a pharmaceutical composition containing the antibody as an active component.

## Description

### [Technical Field]

The present invention relates to an antibody that recognizes an antigen that is specifically presented on a cancer stem cell, and a pharmaceutical composition comprising the antibody, particularly a pharmaceutical composition and a use thereof for treating cancer.

### [Background Art]

The anti-cancer agents which have been developed so far do not have sufficient therapeutic effects, with very low probability of complete cure of cancer. The reason includes that conventional therapeutic agents could not selectively target the cells that underlie cancer tissue. Recently, the presence of cancer stem cells has been reported as such "cells that underlie cancer tissue". The cancer stem cells are considered to be responsible cells being involved in cancer development, recurrence and metastasis. Therefore, it can be expected that by targeting a cancer stem cell, it will be possible to effectively suppress cancer proliferation, recurrence and metastasis. That is, it is an important subject for cancer-medicine to develop a cancer stem cell-detecting technique and novel cancer stem cell-targeting therapeutic agent.

Several genes have been reported as genes which are specifically expressed in cancer stem cells (cancer stem cell antigens) (e.g., Patent Documents 1-3, etc.). Partial peptides of the proteins expressed by these genes are presented as antigens on cell surface. Therefore, it has been attempted to develop a cancer vaccine immunotherapy that is targeted to a cancer stem cell by exploiting such peptides. In order that a cancer vaccine therapy which uses a cancer stem cell antigen-derived peptide exhibits an effect, it is necessary to induce a number of vaccine peptide-specific T cells within patient's body. However, it has been known that it is difficult to induce many T cells in terminal stage cases where immunity is lowered.

### [Prior Art Documents]

### [Patent Documents]

[Patent Document 1] International Patent Application WO 2010/050190
[Patent Document 2] International Patent Application WO 2015/050259
[Patent Document 3] International Patent Application WO 2016/047715

### [Summary of the Invention]

### [Problems to be Solved by the Invention]

The present invention relates to an antibody that recognizes an antigen that is specifically presented as an antigen on a cancer stem cell, and a pharmaceutical composition comprising the antibody, particularly a pharmaceutical composition and a use thereof for treating cancer, a method for treating cancer by targeting cancer stem cells, and the like.

### [Means for Solving the Problems]

In developing a cancer vaccine immunotherapy that is targeted to cancer stem cell which is responsible for the tumorigenicity and and drug resistance of cancer, the present inventors have focused on that a vaccine peptide-specific T cell recognizes a peptide presented on the surface of a cancer cell by HLA-Class I molecules via T-cell receptor, and kills the cancer cell. The present inventors have therefore considered that by generating an antibody that imitates the specificity of this T-cell receptor, it may be possible to practice an immunotherapy that is targeted to a cancer stem cell antigen even in an immunocompromised case. The present inventors thus continued developing such antibody, and successfully separated an antibody fragment (scFv) that recognizes a complex of a cancer stem cell antigenic peptide and a Class I HLA using a phage-display antibody library from human peripheral blood. The present inventors have then obtained a new knowledge that these antibodies exhibited a complement- and antibody-dependent cytotoxic activity against cancer cell lines when being converted to human IgG1 type, further continued the study and as a result completed the present invention.

Accordingly, the present invention relates to those held hereinbelow:
[1] A multispecific antibody having an antigen-binding site that recognizes a complex of an antigenic peptide derived from a protein selected from a group consisting of DNAJB8 protein, BORIS sf6 protein and FAM83B protein and an MHC molecule.
[2] The multispecific antibody according to [1], further having an antigen-binding site that recognizes CD3.
[3] The multispecific antibody according to [1] or [2], wherein the antigenic peptide is a peptide consisting of an amino acid sequence set forth in SEQ ID NOs: 5, 23 or 6.
[4] The multispecific antibody according to any one of [1] to [3], wherein the antigen-binding site that recognizes a complex of an antigenic peptide derived from a protein selected from a group consisting of DNAJB8 protein, BORIS sf6 protein and FAM83B protein and an MHC molecule comprises an amino acid sequence set forth in any one of SEQ ID NOs: 7-14 and 24-38, or an amino acid sequence set forth in any one of the amino acid SEQ ID NOs: 7-14 and SEQ ID NOs: 24-38 in which one or two amino acid(s) are substituted.
[5] The multispecific antibody according to any one of [1] to [4], further comprising a CD80 region.
[6] A pharmaceutical composition comprising an antibody that recognizes a complex of an antigenic peptide derived from a protein selected from a group consisting of DNAJB8 protein, BORIS sf6 protein and FAM83B protein and an MHC molecule.
[7] The pharmaceutical composition according to [6], for preventing and/or treating cancer.
[8] The pharmaceutical composition according to [6] or [7], wherein the MHC molecule is an HLA molecule.
[9] The pharmaceutical composition according to any one of [6] to [8], wherein the antigenic peptide consists of an amino acid sequence set forth in SEQ ID NOs: 5, 23 or 6.
[10] The pharmaceutical composition according to any one of [6] to [9], wherein the antigen-binding site of the antibody comprises an amino acid sequence set forth in any one of SEQ ID NOs: 7-14 and 24-38.
[11] The pharmaceutical composition according to any one of [6] to [10], wherein the antibody is the multispecific antibody according to any one of [1] to [5].

### [Effects of the Invention]

According to the present invention, it becomes possible to practice an immunotherapy that is targeted to a cancer stem cell for a patient with an intractable cancer and sarcoma which shows resistance against existing therapies. Moreover, unlike conventional cancer vaccine immunotherapy, it does not require inducing cytotoxic T cells, and therefore does not require a high immunity which had been considered to be essential for cancer vaccine therapy. Furthermore, because genetical modification of cells, which was necessary for an adoptive immunotherapy of T-cell receptor-expressing T cell, is no longer required, the large preparation of cells (≥ 10¹⁰) which was necessary for the adoptive immunotherapy is also no longer required. Moreover, since the antibody of the present invention has a higher affinity than that of T-cell receptor, it can be expected to have a high drug efficacy as an anti-cancer agent.

### [Brief Description of Drawings]

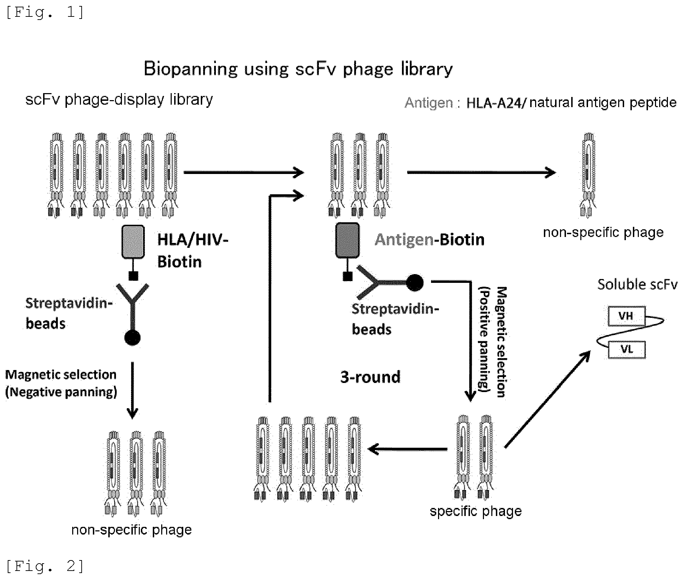

[Fig. 1] A schematic diagram showing an outline of the screening for an scFv antibody by phage-display method. The scFv phage-display library is first screened for whether it reacts to a complex of HLA and HIV, and those which react are removed as nonspecific antibody phages. Then those which react to HLA-A24/natural antigenic peptide complex are selected as specific antibody phages, those which do not react are selected as nonspecific antibody phages; this process is repeated three times. Soluble scFvs are obtained from the specific antibody phages.

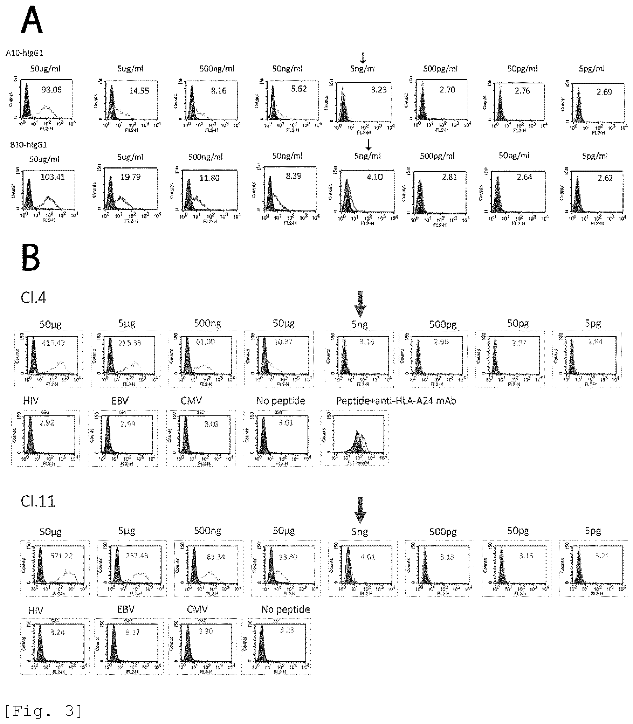

[Fig. 2] A diagram showing the results of the peptide titration assay. It is shown that binding is not detected at concentrations below 5ng/mL (arrows) for all antibodies. Therefore, the reaction detection threshold is considered to be 5ng/mL.

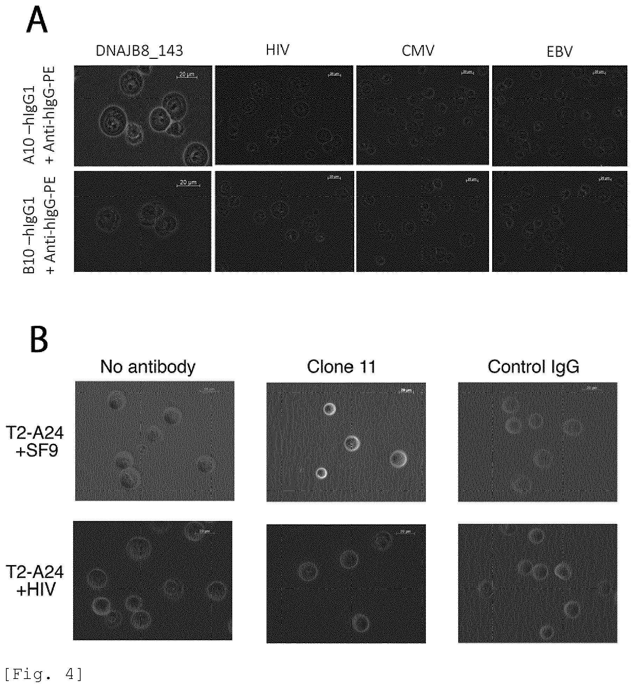

[Fig. 3] Photographs showing that the peptide-pulsed T2-A24 cells are reacted to the antibodies of the present disclosure. (A) Photographs showing the cases in which Clone A10 and Clone B10 are used as antibodies, and DNAJB8-143 is used as the natural antigenic peptide, respectively. (B) Photographs showing the cases in which Clone 11 is used as the antibody, and SF9 is used as the natural antigenic peptide, respectively. It is observed that, when a cancer stem cell antigenic peptide was pulsed, the antibodies of the present invention are bound to the periphery of the cell, which are stained in red.

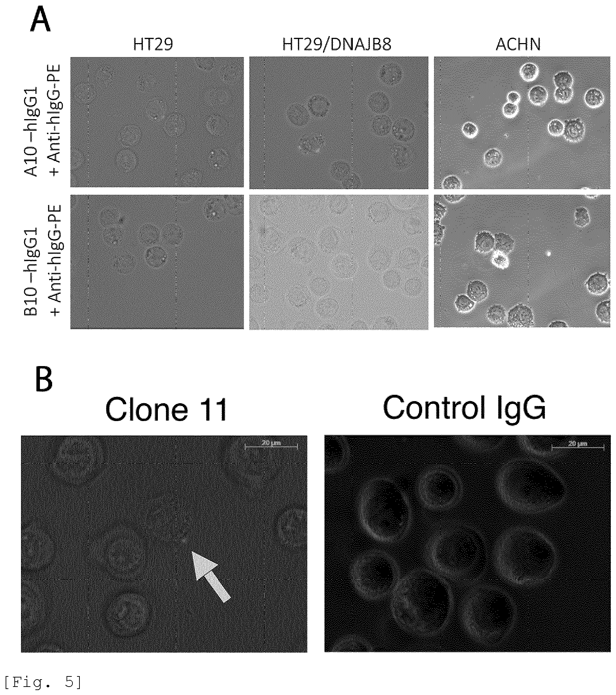

[Fig. 4] Photographs showing that the antibodies of the present disclosure are reacted to the cancer cells which endogenously present natural antigenic peptides. (A) Photographs showing the cases in which Clone A10 and Clone B10 are used as the antibodies. (B) Photographs showing the cases in which Clone 11 is used as the antibody. It is observed that antibodies are bound to parts of plasma membrane of the cells which express HLA-A24 and DNAJB8 or FAM83B.

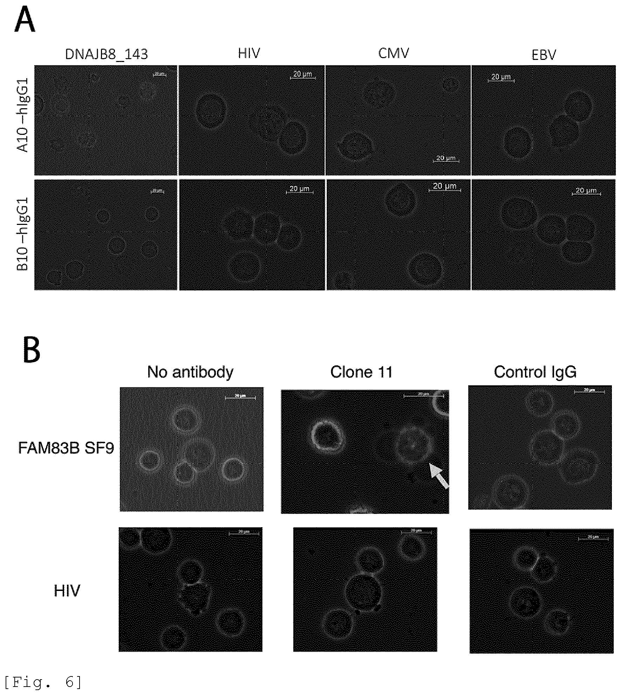

[Fig. 5] Photographs showing observation of cytotoxic activity upon reacting the antibodies of the present disclosure to the peptide-pulsed T2-A24 cells. (A) Photographs showing the cases in which Clone A10 and Clone B10 are used as the antibodies, and DNAJB8-143 is used as the natural antigenic peptide, respectively. (B) Photographs showing the cases in which Clone 11 is used as the antibody, and SF9 is used as the natural antigenic peptide, respectively. It is shown that the complements are bound to the periphery of the cancer stem cell antigenic peptide-pulsed cell, which are stained in red, and that the nuclei are stained in blue by DAPI flowed into the cells through the broken plasma membrane.

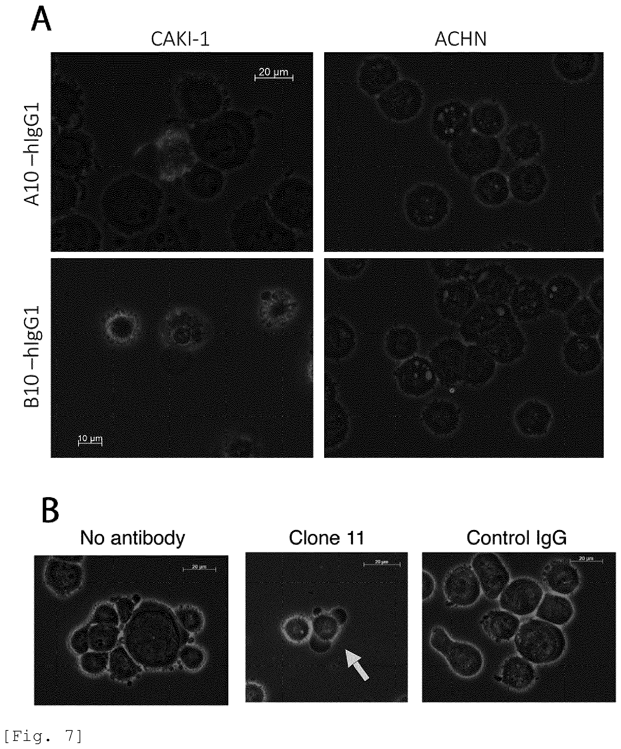

[Fig. 6] Photographs showing observation of cytotoxic activity upon reacting the antibodies of the present disclosure to the cancer cells which endogenously present natural antigenic peptides. (A) Photographs showing the cases in which Clone A10 and Clone B10 are used as the antibodies. (B) Photographs showing the cases in which Clone 11 is used as the antibody. It is shown that the complements, which are stained in red, are bound to the periphery of the cells which express HLA-A24 and DNAJB8 or FAM83B, and that the nuclei are stained in blue by DAPI flowed into the cells through the broken plasma membrane.

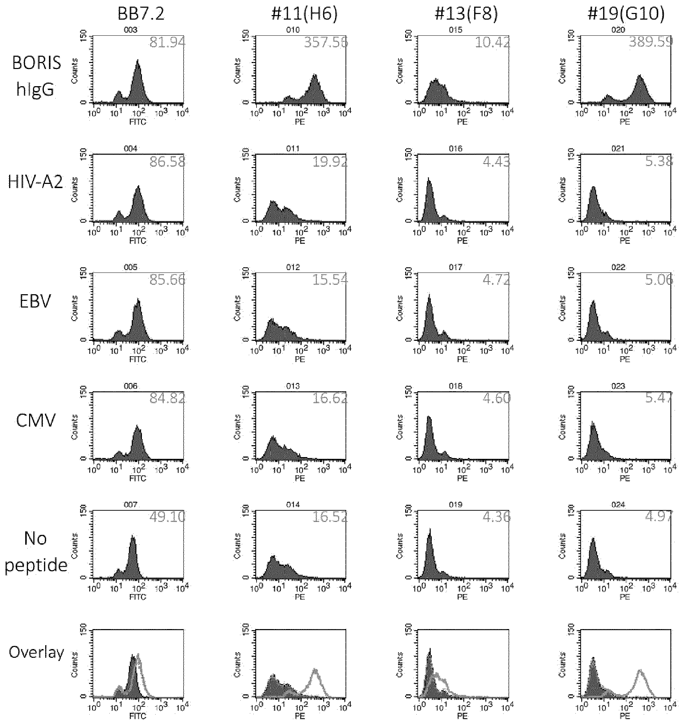

[Fig. 7] A diagram showing the results of FACS analysis for the reactivity of the antibodies of the present disclosure that have been converted from scFv- to hIgG1-type to HLA-A02/LV9 peptide complex. All Clone 11, Clone 13 and Clone 19 antibodies exhibited a high reactivity at the concentration of 200 µg/mL, though Clone 19 showed the highest reactivity.

### [Mode for Carrying Out the Invention]

Hereinbelow, the present invention will be described in detail.

Unless otherwise defined herein, all technical and scientific terminology used herein have the same meanings as those usually understood by a skilled artisan. All patents, applications, and other publications and information cited herein are herein incorporated by reference in their entirety. If there are any conflicts between the publication cited herein and the description of the present specification, the description of the present specification shall control.

In the present disclosure, an "epitope peptide" or "antigenic peptide" means a peptide which is bound by a Major Histocompatibility Complex (MHC (in human, human leukocyte antigen (HLA))) molecule and presented as an antigen on a cell surface, and which has an antigenicity (i.e., which is capable of being recognized by a T cell). Epitope peptides include CTL epitope peptide, which is an epitope peptide that is bound and presented as an antigen by a Class I MHC, and recognized by a CD8-positive T cell; and Helper epitope peptide, which is an epitope peptide that is bound and presented as an antigen by a Class II MHC, and recognized by a CD4-positive T cell.

Among the epitope peptides, a peptide that is derived from a protein specifically or excessively expressed in a tumor cell is referred to as a tumor antigenic peptide. Similarly, a peptide that is derived from a protein specifically or excessively expressed in a cancer stem cell is called a cancer stem cell antigenic peptide. An antigen presentation is an event in which a peptide that is present within a cell is bound by an MHC and this MHC/antigenic peptide complex is localized to cell surface. It is known that an antigen that is presented on the cell surface will be recognized by T cells, etc., and will subsequently activate cellular immunity and humoral immunity. An antigen that is presented by a Class I MHC will activate cellular immunity and be recognized by T-cell receptor of a naive T cell at the same time, inducing the naive T cell to become a CTL, which has a cytotoxic activity. Therefore, in an immunotherapy, a peptide that is bound and presented as an antigen by a Class I MHC is generally used as a tumor antigenic peptide.

Many of the peptides that bind to MHC are known to have a certain characteristic. In the present disclosure, this characteristic is referred to as a "binding motif". The type of the binding motif of a certain peptide to which a certain MHC would bind are known in the art. For instance, the binding motif of HLA-A24, a type of human HMC, comprises tyrosine, phenylalanine, methionine or tryptophan as the second last amino acid from the N-terminal, and comprises leucine, isoleucine or phenylalanine as the C-terminal amino acid. The binding motif of HLA-A02 comprises leucine, isoleucine or methionine as the second last amino acid from the N-terminal, and/or comprises valine, leucine or isoleucine as the C-terminal amino acid.

In the present disclosure, a "natural peptide" refers to a peptide which is actually presented on the cell surface as an antigen. A "natural antigenic peptide" refers to a natural peptide for which its antigenicity has been confirmed. By isolating a complex of a natural antigenic peptide and an MHC molecule from a cancer cell and determining the sequence of the natural antigenic peptide and its origin, a candidate cell surface antigen that will be recognized by an antibody of the present disclosure can be screened.

In the present disclosure, a "tumor" includes benign tumors and malignant tumors (cancer, malignant neoplasms). A cancer includes hematopoietic tumors, epithelial malignant tumors (carcinomas) and non-epithelial malignant tumors (sarcomas).

DNAJB8 is a gene which encodes a DNAJ/HSP40 family member protein of 26kD. Although it is known to be highly expressed in testis, no detailed reports have been made about its localization and functions. Many of DNAJ/HSP40 family members have an N-terminal J domain. It is considered that this J domain binds to HSP70 and stimulates ATP hydrolysis, resulting in a structural change in the substrate binding region of the HSP70 and thus controlling the activity of the HSP70. The HSP40 itself also possesses a peptide binding region, and there are some which have a function of delivering a peptide to HSP70.

FAM83B (family with sequence similarity 83, member B) is a gene which encodes a protein that is presumed to function in the epidermal growth factor receptor (EGFR) signaling pathway, although the detail of its function has yet to be known. It is known that its expression is remarkably elevated in a cancerous part such as a breast cancer, cervical cancer, lung cancer, thyroid cancer, colorectal cancer, testicular tumor and ovarian cancer, as compared to a corresponding non-cancerous part, indicating that it is a potential oncogene that is deeply involved in cancer development, formation and proliferation.

BORIS (Brother of the Regulator of Imprinted Sites) gene is a paralogue of a CTCF gene which is also referred to as 11-zinc finger protein, having 11 zinc finger regions between two coding regions that encode two peptides of N-terminal peptide region and C-terminal peptide region. BORIS is not only known to function as a general transcription factor such as a repressor and an activator in the expression of various genes, but is also known to be expressed in various tumor cells including cancer stem cells, in particular. BORIS can be classified into six subfamilies (sf1 to sf6) according to the sequence of the C-terminal peptide region. Therefore, the sequence of the C-terminal peptide region of each subfamily is a sequence which is unique to that subfamily (e.g., the unique sequence of the BORIS sf6 is shown as the amino acid sequence set forth in SEQ ID NO: 22), which is highly conserved among the isoforms which belong to the same subfamily. Moreover, it has been reported that BORIS is not expressed in a normal tissue except for in testis.

In the present disclosure, when a gene is written simply by a gene name (e.g., "DNAJB8"), it means a gene having a known nucleotide sequence expressed by that gene name, unless otherwise described. It typically indicates a cDNA or mRNA sequence, though being not limited thereto unless it can be recognized by a skilled artisan as the sequence of that gene. For instance, examples of preferred genes and nucleotide sequences thereof in the present disclosure include the following genes expressed by the sequences described below. However, the genes of the present disclosure also include a sequence which can be recognized as the sequence of that gene in a similar way to these genes, such as their genetic polymorphisms (e.g., SNPs, etc.).
DNAJB8: Gene accession No. NM_153330 (SEQ ID NO: 1)
FAM83B: Gene accession No. NM_001010872 (SEQ ID NO: 3)

Accordingly, an mRNA which is a product of gene expression may simply be described by the gene name.

In the present disclosure, when a gene is written with an additional expression "protein" (e.g., "DNAJB8 protein"), it means a protein encoded by that gene, an isoform thereof and a homologue thereof. The isoform includes, e.g., a splicing variant, and a variant based on individual differences, such as SNP. Specifically, the isoform includes: (1) a protein which consists of an amino acid sequence having 90 % and more, preferably 95 % or more, further preferably 98 % or more homology to the protein that is encoded by the gene; and (2) a protein which consists of an amino acid sequence in which one or more, preferably from one to several amino acid(s), further preferably 1 to 10, 1 to 5, 1 to 3, or 1 or 2 amino acid(s) are substituted, deleted, added or inserted in the amino acid sequence of the protein that is encoded by that gene.

In the present disclosure, when an antigenic peptide that is derived from a certain protein is referred to (e.g., an "antigenic peptide derived from DNAJB8 protein"), it refers to a partial peptide which consists of a sequence of a consecutive part of the amino acid sequence that constitutes the certain protein and which possesses the properties of the above-described antigenic peptide.

### <1> Antibodies of the present disclosure

One aspect of the present disclosure relates to an antibody that specifically recognizes a complex of an antigenic peptide and an MHC molecule, wherein the antigenic peptide is derived from a protein which is an expression product of a gene that is specifically expressed in a cancer stem cell such as, e.g., DNAJB8, FAM83B, BORIS, PVT1, ASB4 and LIN28B. Such antibody has, in principle, an antigen-binding site that recognizes the above-described complex of the antigenic peptide and MHC molecule.

In the present disclosure, an "antibody" means a protein which has an antigen-binding site and which has a property of binding to a molecule that is recognized by such antigen-binding site. It is typically an immunoglobulin, though not being limited thereto. Accordingly, the antibody of the present disclosure includes not only an immunoglobulin molecule, but also a functional fragment of an antibody that can be generated from the antigen-binding site of the immunoglobulin (an antigen-binding fragment), etc. Such antigen-binding fragment typically includes a F(ab')₂ fragment, Fab' fragment, Fab fragment, Fv fragment and rIgG fragment, as well as an scFv, dsFv, diabodies and sc(Fv)₂, etc. For instance, these fragments may be linked by a disulfide bond in the constant region and hinge region, or may be single-chained by binding respective regions by linkers (scFv). In a preferred embodiment, the antibody of the present disclosure is a single-chain antibody (scFv). In another preferred embodiment, the antibody of the present disclosure is an IgG1-type antibody. The antibody of the present disclosure also includes a multimer of the immunoglobulin and its functional fragments (e.g., a dimer, trimer, tetramer or polymer).

The antibody of the present disclosure is not particularly limited as long as it can recognize a complex of a cancer stem cell antigenic peptide and an MHC molecule that is present on the surface of a cancer stem cell, and may be a polyclonal or a monoclonal antibody. Moreover, antibody of the present disclosure is not particularly limited as long as it has an antigen-binding site that recognizes the complex of the cancer stem cell antigenic peptide and the MHC molecule. For instance, it may have a binding region, etc., for binding to another antigen-binding site or to another protein. Accordingly, in one certain embodiment, the antibody of the present disclosure may be a multispecific antibody. In a preferred embodiment, the antibody of the present disclosure is a bispecific antibody.

Because the antibody of the present disclosure is capable of recognizing and binding to a complex of a cancer stem cell antigenic peptide and an MHC molecule which is present on the surface of cancer stem cell, it can be used for various applications. In one embodiment, the antibody of the present disclosure can be used in detection of cancer stem cells. In another embodiment, the antibody of the present disclosure can be used in treatment of cancer stem cells (i.e., treatment of cancer). When the antibody of the present disclosure is used in treatment of cancer stem cells, it may be carried out though a method utilizing either cellular immunity or humoral immunity. Embodiments which utilize cellular immunity include, for example, an adoptive cellular immunotherapy using a chimeric antigen receptor (CAR)-introduced T cell (CAR-T) in which an antigen-binding site carried by the antibody of the present disclosure has been integrated. Embodiments which utilize humoral immunity include, for example, a method which utilizes the antibody-dependent cytotoxic (ADCC) activity or complement-dependent cytotoxic (CDCC) activity of the antibody. Accordingly, in a preferred embodiment, the antibody of the present disclosure has an ADCC activity and/or CDCC activity. Moreover, in another embodiment, the antibody of the present disclosure encompasses a chimeric antigen receptor (CAR) in which an antigen-binding site that recognizes a complex of a cancer stem cell antigenic peptide and an MHC molecule has been integrated.

The antibody of the present disclosure, as mentioned above, has an antigen-binding site that specifically recognizes a complex of an antigenic peptide and an MHC molecule, wherein the antigenic peptide is derived from a protein which is an expression product of a gene that is specifically expressed in a cancer stem cell. Any methods known in the art may be used for screening for an antigen-binding site that recognizes a certain antigen or for an antibody that has such antigen-binding site. Such methods include such as, for example, the phage-display method.

Any known gene which has been known in the art to be specifically expressed in a cancer stem cell may be used as the gene that is specifically expressed in a cancer stem cell. Such genes include, such as, for example, DNAJB8, FAM83B, BORIS, PVT1, ASB4 and LIN28B. In a preferred embodiment, the gene that is specifically expressed in a cancer stem cell is DNAJB8, FAM83B or BORIS sf6.

The antigenic peptide is not particularly limited as long as it binds to an MHC. Each of the peptides which bind to an MHC has different characteristics according to its corresponding MHC type. For example, in a case of an HLA (human MHC), a peptide that binds to a Class I HLA is about 8 to 14 amino acid long, preferably about 8 to 10 amino acid long, whereas a Class II HLA binds to a peptide that is 10 amino acid long or longer, for example, about 10 to 30 amino acid long. Moreover, among the Class I HLAs, a peptide which binds to HLA-A02, for instance, has a binding motif in which the N-terminal second last amino acid is leucine, isoleucine or methionine, and/or in which the C-terminal amino acid is valine, leucine or isoleucine; a peptide which binds to HLA-A24 has a binding motif in which the N-terminal second last amino acid is tyrosine, phenylalanine, methionine or tryptophan, and/or in which the C-terminal amino acid is leucine, isoleucine or phenylalanine.

The antigenic peptide may be determined by predicting an MHC-restricted peptide from the full length of the protein based on the binding motif, or may be determined by identifying the natural peptide that is actually presented as an antigen. The method of identifying the natural peptide may be any of the methods known in the art or a combination thereof, including, for example, the methods described in the above-described Patent Document 2. Since the antibody of the present disclosure recognizes an antigenic peptide that is presented as an antigen on the cell surface, the antigenic peptide preferably is a natural peptide. By the present inventors, DNAJB8-143 (AFMEAFSSF (SEQ ID NO: 5)) has been identified as a DNAJB8 natural peptide in a human cancer stem cell; SF9 (SYQPNENKF (SEQ ID NO: 6)) as a FAM83B natural peptide; and LV9 (LLFIGTIKV (SEQ ID NO: 23)), which is a partial polypeptide of BORIS sf6 unique sequence (SEQ ID NO: 22), as a natural peptide, respectively. Accordingly, in a more preferred embodiment, the antigenic peptide is DNAJB8-143, SF9, or LV9.

In a preferred embodiment of the present disclosure, the MHC is an HLA. In a more preferred embodiment, the MHC is a Class I HLA. In a further preferred embodiment, the MHC is HLA-A02. In another further preferred embodiment, the MHC is HLA-A24.

The antibody of the present disclosure is, as mentioned above, not particularly limited as long as it is capable of specifically recognizing a complex of an antigenic peptide and an MHC molecule. It may be an antibody that further recognizes and binds to another molecule. Accordingly, in a preferred embodiment, the antibody of the present disclosure may have a binding region to another molecule in addition to the antigen-binding site that recognizes the complex of the cancer stem cell antigenic peptide and the MHC molecule. Such binding region typically is an antigen-binding site, but is not limited thereto. For instance, it may be a ligand for a cell surface receptor, etc. In a preferred embodiment, the antibody of the present disclosure is an antibody which specifically binds to two or more molecules, namely a multispecific antibody. In the present disclosure, a "multispecific antibody" means an antibody that has at least one antigen-binding site and specifically binds to two or more molecules. Accordingly, for instance, when a bispecific antibody of the present disclosure is referred to, it means an antibody that has at least one antigen-binding site and specifically binds to two molecules, wherein the at least one antigen-binding site is an antigen-binding site that recognizes a complex of a cancer stem cell antigenic peptide and an MHC molecule, and the antibody being capable of further specifically binding to another molecule. The specific binding to another molecule may be achieved by another antigen-binding site, or may be achieved by other methods such as, for example, by a ligand region to another molecule.

When the antibody of the present disclosure is a multispecific antibody, another antigen that is recognized by the antibody of the present disclosure is not particularly limited, though it preferably facilitates the treatment of a cancer stem cell. Such antigen includes such as, for example, proteins expressed on the cell surface of an immunocyte, such as CD3, CD28, CD40, PD1, CTLA4, TIGIT, OX40 and CD137. In a preferred embodiment of the present disclosure, other antigens include T-cell surface proteins such as CD3 or CD28. Accordingly, in a preferred embodiment, the multispecific antibody of the present disclosure has a binding region to CD3 and/or CD28 in addition to the antigen-binding site that recognizes the complex of the cancer stem cell antigenic peptide and the MHC molecule. Such binding region may be an antigen-binding site, or may be CD80, etc. as a ligand to CD28, for example.

By the present inventors, for the above-mentioned natural antigenic peptides DNAJB8-143, SF9, and LV9, antibodies were screened for those which have an antigen-binding site that recognizes a complex of one of these antigenic peptides and HLA-A24 or HLA-A02. Thus, in a preferred embodiment of the present disclosure, the antigen-binding site that recognizes the complex of the cancer stem cell antigenic peptide and the MHC molecule comprises an amino acid sequence set forth in any one of SEQ ID NOs: 7-14 and SEQ ID NOs: 24-38, or an amino acid sequence indicated by GNT, DGT or HDS. In another preferred embodiment of the present disclosure, the antigen-binding site that recognizes the complex of the cancer stem cell antigenic peptide and the MHC molecule may comprise an amino acid sequence set forth in any one of SEQ ID NOs: 7-14 and SEQ ID NOs: 24-38 in which one or two amino acid(s) has/have been substituted. The amino acid substitution preferably is a conservative substitution such as a substitution between acidic amido acids, a substitution between basic amino acids, or a substitution between neutral amino acids.

Typical examples of the antibodies of the present disclosure include:
a specific antibody having an antigen-binding site that recognizes a complex of a cancer stem cell antigenic peptide consisting of the amino acid sequence set forth in SEQ ID NO: 5 (DNAJB8-143) and HLA-A24;
a specific antibody having an antigen-binding site that recognizes a complex of a cancer stem cell antigenic peptide consisting of the amino acid sequence set forth in SEQ ID NO: 6 (SF9) and HLA-A24;
a bispecific antibody having an antigen-binding site that recognizes a complex of a cancer stem cell antigenic peptide consisting of the amino acid sequence set forth in SEQ ID NO: 5 (DNAJB8-143) and HLA-A24, and an antigen-binding site that recognizes CD3;
a bispecific antibody having an antigen-binding site that recognizes a complex of a cancer stem cell antigenic peptide consisting of the amino acid sequence set forth in SEQ ID NO: 6 (SF9) and HLA-A24, and an antigen-binding site that recognizes CD3;
a trispecific antibody having an antigen-binding site that recognizes a complex of a cancer stem cell antigenic peptide consisting of the amino acid sequence set forth in SEQ ID NO: 5 (DNAJB8-143) and HLA-A24, an antigen-binding site that recognizes CD3, and an antigen-binding site that recognizes CD28;
a trispecific antibody having an antigen-binding site that recognizes a complex of a cancer stem cell antigenic peptide consisting of the amino acid sequence set forth in SEQ ID NO: 6 (SF9) and HLA-A24, an antigen-binding site that recognizes CD3, and an antigen-binding site that recognizes CD28;
a trispecific antibody having an antigen-binding site that recognizes a complex of a cancer stem cell antigenic peptide consisting of the amino acid sequence set forth in SEQ ID NO: 5 (DNAJB8-143) and HLA-A24, an antigen-binding site that recognizes CD3, and CD80 region; and
a trispecific antibody having an antigen-binding site that recognizes a complex of a cancer stem cell antigenic peptide consisting of the amino acid sequence set forth in SEQ ID NO: 6 (SF9) and HLA-A24, an antigen-binding site that recognizes CD3, and CD80 region, and the like.

Other typical examples of the antibodies of the present disclosure include:
a specific antibody having an antigen-binding site that recognizes a complex of a cancer stem cell antigenic peptide consisting of the amino acid sequence set forth in SEQ ID NO: 23 (LV9) and HLA-A02;
a bispecific antibody having an antigen-binding site that recognizes a complex of a cancer stem cell antigenic peptide consisting of the amino acid sequence set forth in SEQ ID NO: 23 (LV9) and HLA-A02, and an antigen-binding site that recognizes CD3;
a trispecific antibody having an antigen-binding site that recognizes a complex of a cancer stem cell antigenic peptide consisting of the amino acid sequence set forth in SEQ ID NO: 23 (LV9) and HLA-A02, an antigen-binding site that recognizes CD3, and an antigen-binding site that recognizes CD28; and
a trispecific antibody having an antigen-binding site that recognizes a complex of a cancer stem cell antigenic peptide consisting of the amino acid sequence set forth in SEQ ID NO: 23 (LV9) and HLA-A02, an antigen-binding site that recognizes CD3, and CD80 region, and the like.

The antibody of the present disclosure preferably comprises at least one of the complementarity-determining region (CDR) 1, the complementarity-determining region (CDR) 2 and the complementarity-determining region (CDR) 3 in the antigen-binding site, and comprises at least one of the complementarity-determining region (CDR) 1, the complementarity-determining region (CDR) 2 and the complementarity-determining region (CDR) 3 in both heavy and light chains.

In a preferred embodiment, the antibody of the present disclosure particularly comprises, in the antigen-binding site, the amino acid sequence set forth in SEQ ID NO: 7 and/or SEQ ID NO: 8 as the complementarity-determining region (CDR) 3. In another preferred embodiment, it particularly comprises, in the antigen-binding site, the amino acid sequence set forth in SEQ ID NO: 9 and/or SEQ ID NO: 10 as the complementarity-determining region (CDR) 3. Further, in another preferred embodiment, it particularly comprises, in the antigen-binding site, the amino acid sequence set forth in SEQ ID NO: 11 and/or SEQ ID NO: 12 as the complementarity-determining region (CDR) 3. Further, in another preferred embodiment, it particularly comprises, in the antigen-binding site, the amino acid sequence set forth in SEQ ID NO: 13 and/or SEQ ID NO: 14 as the complementarity-determining region (CDR) 3.

Furthermore, in a preferred embodiment, the antibody of the present disclosure may, in its antigen-binding site, comprise an amino acid sequence set forth in any one of amino acid SEQ ID NOs: 7-14 in which one or two amino acid(s) have(has) been substituted.

Moreover, in a preferred embodiment, the antibodies of the present disclosure particularly comprises, in the antigen-binding site, the amino acid sequence set forth in SEQ ID NO: 24 and/or SEQ ID NO: 27 as the complementarity-determining region (CDR) 1, the amino acid sequence set forth in SEQ ID NO: 25 and/or an amino acid sequence indicated by GNT as the complementarity-determining region (CDR) 2, and the amino acid sequence set forth in SEQ ID NO: 26 and/or SEQ ID NO: 28 as the complementarity-determining region (CDR) 3. In another preferred embodiment, it particularly comprises, in the antigen-binding site, the amino acid sequence set forth in SEQ ID NO: 29 and/or SEQ ID NO: 32 as the complementarity-determining region (CDR) 1, the amino acid sequence set forth in SEQ ID NO: 30 and/or an amino acid sequence indicated by DGT as the complementarity-determining region (CDR) 2, and the amino acid sequence set forth in SEQ ID NO: 31 and/or SEQ ID NO: 33 as the complementarity-determining region (CDR) 3. Furthermore, in another preferred embodiment, it particularly comprises, in the antigen-binding site, the amino acid sequence set forth in SEQ ID NO: 34 and/or SEQ ID NO: 37 as the complementarity-determining region (CDR) 1, the amino acid sequence set forth in SEQ ID NO: 35 and/or an amino acid sequence indicated by HDS as the complementarity-determining region (CDR) 2, and the amino acid sequence set forth in SEQ ID NO: 36 and/or SEQ ID NO: 38 as the complementarity-determining region (CDR) 3.

Furthermore, in a preferred embodiment, the antibody of the present disclosure may, in its antigen-binding site, comprise an amino acid sequence set forth in any one of SEQ ID NO: 24-38 in which one or two amino acid(s) have(has) been substituted.

Besides, in the antibody of the present disclosure, CDR3 is particularly important for antigen-binding among three CDRs.

### <2> Pharmaceutical compositions of the present disclosure

One aspect of the present disclosure relates to a pharmaceutical composition comprising an antibody of the present disclosure. As mentioned above, since the antibody of the present disclosure is capable of specifically recognizing a cancer stem cell antigenic peptide that is presented as an antigen on the surface of a cancer stem cell, it can be used as an active ingredient in a pharmaceutical composition for various applications. Accordingly, any antibody which has been mentioned in detail in <1> above may be used as the antibody of the present disclosure which is contained as an active ingredient in the pharmaceutical composition of the present disclosure.

The pharmaceutical composition of the present disclosure comprises an antibody of the present disclosure as an active ingredient. The pharmaceutical composition of the present disclosure may not only be utilized as a treatment agent for treating cancer stem cells (i.e., a cancer therapeutic agent), but may also be used, for example, as a detecting agent for a cancer stem cell and as a companion diagnostic for determining the effectiveness of a cancer vaccine immunotherapy to a patient who is the subject of the treatment.

As mentioned above, in one preferred embodiment, the antibody of the present disclosure has an ADCC activity and/or CDCC activity. Namely, the antibody of the present disclosure can exert a cytotoxic activity on a cancer stem cell by recognizing a cancer stem cell antigenic peptide presented on the surface of the cancer stem cell and binding to the surface of the cancer stem cell. Therefore, in a preferred embodiment, the pharmaceutical composition of the present disclosure is a pharmaceutical composition for preventing/treating cancer.

Here, "prevention" of cancer includes not only preventing a patient from being affected by cancer, but also includes preventing recurrence in a patient who has undergone resection of the primary lesion of a tumor, and preventing metastasis of a tumor which was failed to be completely removed by cancer therapies such as surgery, radiotherapy or chemotherapy. Moreover, "treatment" of cancer includes not only curing a cancer or improving symptoms to reduce a cancer, but also includes preventing its progression by suppressing proliferation of cancer cells, tumor expansion or cancer cell metastasis from the primary lesion.

The pharmaceutical composition of the present disclosure may be administered to any individual organism which may have a tumor. The subject of administration is preferably an individual of human or non-human mammal (e.g., a rodent such as a mouse, rat, guinea pig or hamster, a primate such as a chimpanzee, an artiodactyl such as a cow, goat or sheep, a perissodactyl such as a horse, or a rabbit, dog, cat, etc.), more preferably a human individual.

When the pharmaceutical composition of the present disclosure is used as a tumor detection agent, the subject for detection can be a cell population derived from any biological sample obtained from the above-described individual organism, preferably a cell population derived from any biological sample obtained from a human, more preferably a cell population comprising cells derived form a tissue excluding testis in which it has been confirmed that either DNAJB8, FAM83B or BORIS sf6 is scarcely expressed in the cells of the tissue, for example, from one or more biological sample(s) selected from a group consisting of heart, brain, placenta, lung, liver, skeletal muscle, kidney, pancreas, spleen, thymus, prostate, testis, ovary, small intestine, large intestine and blood.

As mentioned above, one preferred embodiment of the antibody of the present disclosure has an antigen-binding site that recognizes a complex of a cancer stem cell antigenic peptide derived from DNAJB8 or FAM83B protein and HLA-A24. Another preferred embodiment of the antibody of the present disclosure has an antigen-binding site that recognizes a complex of a cancer stem cell antigenic peptide derived from BORIS sf6 protein and HLA-A02. Accordingly, the pharmaceutical composition of the present disclosure can particularly suitably be used in a subject who has a cancer in which either DNAJB8, FAM83B or BORIS sf6 is expressed. Moreover, it can particularly suitably be used in a subject who has HLA-A24 or HLA-A02 as the HLA. In specific, it can be used for prophylaxis or treatment of a cancer (tumor) such as, e.g., colorectal cancer, lung cancer, breast cancer, myeloma, oral cancer, pancreas cancer, skin cancer and prostate cancer.

In recent years, it has become clear that cancer cells escape elimination by immune system by shielding an attack by immunocytes, and that such shield make use of a mechanism called "immune checkpoint", which is inherent in cells for suppressing excessive autologous immune reaction and damages to normal tissue. Therefore, by suppressing the immune checkpoint function in a cancer cell, it is possible to make the attack by immunocytes effective. The pharmaceutical composition of the present disclosure, in one embodiment, exerts an antitumor effect by utilizing an immunocyte which is capable of injuring a cancer stem cell to which the antibody, the active ingredient, has been bound. Accordingly, it is considered that the pharmaceutical composition of the present disclosure can exert a higher therapeutic effect by suppressing the immune checkpoint function at the same time. Thus, in a preferred embodiment, the pharmaceutical composition of the present disclosure is used together with an immune checkpoint inhibitor.

In the present disclosure, when an agent A and another agent B are said to be "used together" or "used in combination", it refers to rendering the agent B effective during a period in which the agent A is exerting its effect. Therefore, the agent B may be administered concomitantly with the agent A, or the agent B may be administered after certain interval after administration of the agent A. The agent A and the agent B may be in an identical dosage form, or may be in different dosage forms. Furthermore, the agent A and the agent B may be mixed to form one composition unless either the agent A or the agent B loses its effect.

The immune checkpoint inhibitor which can be used in in the present embodiment may be any agent that has been known as an immune checkpoint inhibitor as long as it does not inhibit the antigen-recognizing ability of the pharmaceutical composition of the present disclosure. Known immune checkpoint inhibitors include, but is not limited to, such as, e.g., anti-PD-1 antibody, anti-PD-Ll antibody, anti-CTLA-4 antibody, anti-TIM-3 antibody, anti-LAG-3 antibody, anti-B7-H3 antibody, anti-B7-H4 antibody, anti-B7-H5 antibody and anti-TIGIT antibody.

The dosage form of the pharmaceutical composition of the present disclosure is not particularly limited, and includes, such as, an oil emulsion (emulsion preparation), a polymer nanoparticle, a liposome preparation, a particulate preparation attached to beads having a diameter of several micrometer, a lipid-bound preparation, a microsphere preparation and a microcapsule preparation.

Methods for administration include any known methods for administration such as intradermal administration, subcutaneous administration, intramuscular administration and intravenous administration. The dosage of the pharmaceutical composition of the present disclosure in a preparation may be appropriately adjusted according to the disease to be treated, the age or weight of the patient, etc., though it is usually between 0.0001 mg and 1000 mg, preferably between 0.001 mg and 1000 mg, more preferably between 0.1 mg and 10 mg, which is preferably administered once in a few days to once in a few months.

Moreover, as mentioned above, it is also possible to treat cancer by adoptive cellular immunotherapy that uses a chimeric antigen receptor (CAR)-introduced T cell (CAR-T) into which an antigen-binding site possessed by the antibody of the present disclosure has been integrated. In the present disclosure, the "chimeric antigen receptor" is a chimeric protein molecule designed to have a single chain antibody (scFv), in which a light chain and a heavy chain of the antibody variable region of an antibody that recognizes a molecule present on the cell surface of a cancer cell, at the N-terminal side, and a CD3ζ chain, among the molecules which constitute a T-cell receptor (TCR)/CD3 complex, at the C-terminal side. When this chimeric antigen receptor recognizes certain antibody in the scFv region, the activation of T cells is initiated via the CD3ζ chain. In order to enhance the activation of T cells, one or more costimulatory molecule (e.g., CD28, 4-1BB, ICOS, etc.) may be integrated between the scFv and the ζ chain. Thus, the antibodies of the present disclosure can be used as the scFv to generate a CAR. The CAR that recognizes a complex of a cancer stem cell antigenic peptide and an MHC is capable of recognizing a cancer stem cell presenting a cancer stem cell antigenic peptide which can be targeted by a CTL and a dendritic cell which presenting a phagocytosed tumor antigenic peptide on Class I MHC, etc. Accordingly, a pharmaceutical composition comprising the above-mentioned CAR-introduced genetically modified T cell (CAR-T) is also encompassed by the pharmaceutical composition of the present disclosure.

### <3> Methods for detecting tumor (examination and diagnostic methods)

One aspect of the present disclosure relates to methods for detecting tumor (examination and diagnostic methods) utilizing the antibodies of the present disclosure.

A method for detection (diagnostic method) of the present disclosure using an antibody of the present disclosure is for detecting, examining or diagnosing the presence or absence or the degree of a cancer (tumor) such as colorectal cancer, lung cancer, breast cancer, myeloma, oral cancer, pancreatic cancer, skin cancer and prostate cancer, typically by collecting the blood of the test subject or collecting a part of the test subject tissue that is suspected to have a tumor by biopsy, etc., and detecting or measuring the amount of cells having a complex of the cancer stem cell antigenic peptide and the MHC molecule contained therein using an antibody of the present disclosure.

A certain embodiment of the method for detecting (examining) of the present disclosure using an antibody of the present disclosure comprises the following steps (a) and (b), and optionally the step (c) :
(a) a step of bringing a biological sample obtained from a test subject into contact with a tumor-detecting agent of the present disclosure;
(b) a step of measuring the amount of cells presenting a complex of a cancer stem cell antigenic peptide and an HLA antigen in the biological sample in reference to the amount of cells to which the above-described tumor-detecting agent has been bound as an index;
(c) a step of determining the presence of a cancer based on the result from (b).

A certain embodiment of the diagnostic method of the present disclosure using an antibody of the present disclosure comprises the steps (a), (b) and (c) as described above.

The biological sample used here includes a sample prepared from a biological tissue (a tissue in which the presence of a cancer cell is suspected and the peripheral tissue thereof, or blood, etc.) of a test subject. Specifically, it includes a sample containing tissue cells collected from such tissue.

The prediction, evaluation, determination or diagnosis of the presence or absence of a tumor can be carried out, for example, by measuring the amount of cells to which an antibody of the present disclosure has been bound in the blood of the test subject or the test subject tissue that is suspected of having a tumor. This can be carried out, in some cases, by providing a standard value such as the level of the cells to which the antibody of the present disclosure has been bound in a corresponding normal tissue, comparing this standard value with the above-mentioned level in the sample obtained from the test subject, and evaluating the difference therebetween.

Here, the comparison of the above-mentioned level between the test subject tissue of the test subject and the corresponding normal tissue can be performed by carrying out the measurement directed to the biological sample of the test subject in parallel with the measurement directed to the biological sample of a healthy subject. If it is not carried out in parallel, a statistical mean or median value of the amount of the cells, to which the antibody of the present disclosure is bound, obtained by measuring under a uniform measuring condition using a multiple (at least 2, preferably 3 or more, more preferably 5 or more) normal tissues can be used for comparison as a value of a healthy subject, i.e., the standard value.

The determination whether the test subject has a cancer or not can be carried out based on an index, for example, that the cells to which the antibody of the present disclosure is bound in the tissue of the test subject is, for example, twice or more or three-times or more as compared to the level of it in a healthy subject.

### <4> Methods for preventing and/or treating cancer

One aspect of the present disclosure relates to a method for preventing/treating cancer in a subject, comprising a step pf administering an effective amount of the antibody or CAR-T cell of the present disclosure to a subject in need thereof.

The "subject" in the present disclosure can be any individual organism as long as it is an individual organism who could have a cancer, though, it is preferably an individual of human and non-human mammal (e.g., a rodent such as a mouse, rat, guinea pig and hamster, a primate such as a chimpanzee, an artiodactyl such as a cow, goat and sheep, a perissodactyl such as a horse, and a rabbit, dog, cat, etc.), and more preferably a human individual. In the present disclosure, the subject may be either healthy or suffering from some diseases. However, when the prevention and/or treatment of cancer is contemplated, the subject typically means a subject who has a cancer or is at a risk of having a cancer. In one embodiment of the present disclosure, the subject is either HLA-A24- or HLA-A02-positive. In a preferred embodiment of the present disclosure, the subject has or is at a risk of having DNAJB8- or FAM83B- or BORIS sf6-positive cancer. In one embodiment of the present disclosure, the subject has or is at a risk of having a cancer which is HLA-A24-positive and DNAJB8- or FAM83B-positive. In another embodiment of the present disclosure, the subject has or is at a risk of having a cancer which is HLA-A02-positive and BORIS sf6-positive.

The antibody and CAR-T cell of the present disclosure to be used in the method of prevention/treatment of the present disclosure include any of those described herein. An effective amount in the present disclosure is an amount which reduces symptoms of a cancer, or delays or halts its progress, preferably an amount which suppresses or cures a cancer. Moreover, an amount which does not cause an adverse effect that exceeds the benefit obtained by the administration is preferable. Such amount can appropriately be determined by *in vitro* examination using a cultured cell, etc., or by an examination in a model animal such as a mouse or rat. Such examination methods are well-known to a skilled artisan. Specific doses of the active ingredient may be determined in view of various conditions associated with the subject in need thereof, for example, the severity of the symptom, the general health condition, age, body weight and gender of the subject, diets, the timing and frequency of administration, concomitant medicaments, the reactivity to the treatment, dosage forms, and the compliance to the treatment, etc.

A specific dose is, for example, in a case of an antibody of the present disclosure, usually between 0.0001 mg and 2000 mg, preferably between 0.001 mg and 2000 mg, which is preferably administered from once a week to once in four weeks. In a case of CAR-T cell of the present disclosure, it is usually between 1 x 10⁴ and 1 x 10⁸ cells, preferably between 1 x 10⁵ and 1 x 10⁷ cells, which is preferably administered from once a week to once in four weeks. Moreover, methods for administration which can be used may be any known appropriate methods such as intradermal administration, subcutaneous administration, intramuscular administration and intravenous administration.

One embodiment of the method for prevention/treatment of the present disclosure further comprises, prior to the administering step, a step of selecting a subject who is HLA-A24- or HLA-A02-positive as the subject for the prevention/treatment. This embodiment of the present disclosure may further comprise, prior to the step of the above-mentioned selecting step, a step of determining the HLA-type pf the subject. The determination of the HLA-type pf the subject can be carried out by any known procedures. Moreover, one embodiment of the method for prevention/treatment of the present disclosure further comprises, prior to the administering step, a step of selecting a subject who has DNAJB8- or FAM83B- or BORIS sf6-positive cancer as the subject for the prevention/treatment. This embodiment of the present disclosure may further comprise, prior to the step of the above-mentioned selecting step, a step of detecting the DNAJB8- or FAM83B- or BORIS sf6-positive cancer in the subject. The detection of the DNAJB8- or FAM83B- or BORIS sf6-positive cancer in the subject can be carried out by a method for detecting tumor as described in the above section <3>. One embodiment of the method for prevention/treatment of the present disclosure further comprises, prior to the administering step, a step of selecting a subject who has a cancer that is HLA-A24-positive and DNAJB8- or FAM83B-positive, or a subject who has a cancer that is HLA-A02-positive and BORIS sf6-positive as the subject for the prevention/treatment. This embodiment of the present disclosure may further comprise, prior to the step of the above-mentioned selecting step, a step of determining the HLA-type pf the subject and a step of detecting the DNAJB8- or FAM83B- or BORIS sf6-positive cancer in the subject.

Hereinbelow, the present invention will be specifically illustrated with examples, though the present invention is not limited by these examples.

### [Examples]

### Example 1. Screening for HLA-A24/natural antigenic peptide complex-specific antibodies

### (1) Generation of antibody-phages

Using a DNAJB8 protein-derived natural antigenic peptide DNAJB8-143 (SEQ ID NO: 5) and a FAM83B protein-derived natural antigenic peptide SF9 (SEQ ID NO: 6) as cancer stem cell antigenic peptides, scFvs that recognize HLA-A24/DNAJB8-143 complex and HLA-A24/SF9 complex were identified using a similar method to the phage-display method described in Tsukahara et al., J Biol Chem., 2014, Aug 8;289(32):22035-47. In the following Examples 1 to 5, the "cancer stem cell antigenic peptides" means DNAJB8-143 and SF9.

Specifically, first, from the scFv library, a phagemid vector was generated in which a DNA encoding for a peptide in which the heavy-chain variable region (VH region) and light-chain variable region (VL region) of an antibody were connected by a linker has been integrated, and used to infect *Escherichia coli.* This was further infected with a helper phage to generate an M13 phage that presents an scFv in which the VH region and the VL region were connected with a linker (the antibody phage).

### (2) Biopanning

Prior to the biopanning, the nonspecifically-bound phages were removed from the phage library using a biotin-modified complex of HLA-A24 and HIV peptide (RYLRDQQLLGI (SEQ ID NO: 15)) and the streptavidin-bound magnetic beads. From the remaining antibody phages, using the biotin-modified HLA-A24/DNAJB8-143 complex or the biotin-modified HLA-A24/SF9 complex and streptavidin-bound magnetic beads, the antibody phages which bind to each complex were screened (positive panning). This step was repeated three times to give candidate antibody phages which specifically bind to the HLA-A24/natural antigenic peptide complex.

### (3) Conversion of scFv antibody to IgG1-type and FACS analysis

The scFv part was separated from the obtained candidate antibody phage, labelled with a FLAG tag at the end of the VL region, and the reactivity to HLA-A24/DNAJB8-143 complex or HLA-A24/SF9 complex was analyzed using FACS. As a result of the analysis, clones with high reactivity were screened, the heavy chain Fc region of human IgG1 (CH2 and CH3 regions, SEQ ID NO: 19) was linked instead of FLAG tag, and the scFv antibodies were converted to IgG1-type. The antibodies converted to IgG1-type (scFv-hIgG1) were used to carry out further FACS analysis, and the clones with particularly high reactivity (DNAJB8-143: Clone A10 and Clone B10, SF9: Clone 4 and Clone 11) were screened.

The sequences of the CDR3 region of these clones were as follows:
Clone A10 VH region: CARVGWDLLGRAFDIW (SEQ ID NO: 7)
Clone A10 VL region: CAAWDDSLSGVVF (SEQ ID NO: 8)
Clone B10 VH region: CARDGEMATVVAGPFDNW (SEQ ID NO: 9)
Clone B10 VL region: CQVWDSSVVF (SEQ ID NO: 10)
Clone 4 VH region: CAKDIGSGWFSMDVW (SEQ ID NO: 11)
Clone 4 VL region: CAKDIGSGWFSMDVW (SEQ ID NO: 12)
Clone 11 VH region: CAKGKYSGSYYALDYW (SEQ ID NO: 13)
Clone 11 VL region: CQSYDSSLSGSVF (SEQ ID NO: 14)

### Example 2. Peptide titration assay

HLA-A24-expressing T2 cell (T2-A24 cell) was pulsed with a natural antigenic peptide at various concentration (50 µg/mL, 5 µg/mL, 500 ng/mL, 50 ng/mL, 5 ng/mL, 500 pg/mL, 50 pg/mL and 5 pg/mL, respectively), and then reacted with an antibody clone obtained in Example 1 to calculate the reaction threshold concentration. Cells that were pulsed with either HIV (SEQ ID NO: 15), EBV (TYGPVFMSL (SEQ ID NO: 16)), or CMV (QYDPVAALF (SEQ ID NO: 17)) peptide at 50 µg/mL were used as negative controls.

The results are shown in Fig. 2. For all clones, the reaction threshold concentration was about 5 ng/mL.

### Example 3. Reactivity to antigen-presenting cells

### (1) Reactivity to the peptide-pulsed T2-A24 cell

The scFv-hIgG1 antibody obtained in Example 2 was examined for whether it actually reacts with a cell that presents an antigen on the cell surface. T2-A24 cell was pulsed with either DNAJB8-143, HIV, CMV or EBV prepared to the final concentration of 50 µg/mL, and then reacted with A10 antibody or B10 antibody obtained in Example 2 at the final concentration of 10 µg/mL, immunostained with anti-hIgG-PE for observation. For Clone 11 antibody, similar observation was carried out except that T2-A24 cell was pulsed with SF9 or HIV prepared to the final concentration of 100 µg/mL.

The results are shown in Fig. 3. It was observed that the scFv-IgG1 antibody was bound to the surface of the T2-A24 cell that had been pulsed with either of the cancer stem cell antigenic peptides.

### (2) Reactivity to cancer cells expressing various proteins

Next, reactivity to the actual cancer cells was examined. The examination was carried out in a similar way to (1) except that a human colon adenocarcinoma cell line HT29 (HLA-A24(+) and DNAJB8(+)), a DNAJB8-overexpressing HT29, and a human renal adenocarcinoma cell line ACHN (HLA-A24(-) and DNAJB8(+)) were used in the examination for A10 antibody and B10 antibody, and that the SP fraction cell of a human colorectal cancer cell line SW480 (HLA-A24(+) and FAM83B (+)) was used in the examination for Clone 11, respectively.

The results are shown in Fig. 4. It was confirmed that, the scFv-hIgG1 antibody was bound to the cell surface of each of the cancer cells in a similar way to T2-A24 cell. Therefore, it was shown that the antibody of the present disclosure is also capable of recognizing endogenously presented natural antigenic peptides.

### Example 4. Validation of complement-dependent cytotoxic (CDCC) activity

### (1) Cytotoxic activity to peptide-pulsed T2-A24 cell

T2-A24 cell was pulsed with a peptide in a similar way to Example 3 (1), then reacted with each of the antibodies prepared to the final concentration of 10 µg/mL, and observed using PE-conjugated anti-C3b antibody and DAPI as fluorescent labels. CDCC activity will be confirmed when the cell periphery is stained in red by the bound C3b and the nucleus is stained in blue by DAPI flowed into the cells through the plasma membrane that was broken by the CDCC activity.

The results are shown in Fig. 5. For all antibodies, CDCC activity to the cancer stem cell antigenic peptide-pulsed T2-A24 was confirmed.

### (2) Cytotoxic activity to cancer stem cells

The examination was carried out in a similar way to Example 4 (1) using the same cells as Example 3 (2) except that a human renal cell Caki-1 (HLA-A24 (+) and DNAJB8 (+)) was used instead of the human colon adenocarcinoma cell line HT29 (HLA-A24 (+) and DNAJB8 (+)).

The results are shown in Fig. 6. In the actual cancer cells, DAPI inflow due to the damage to the plasma membrane was observed in a similar way to that in T2-A24 cell. Thus, CDCC activity was also confirmed to a cancer cell which endogenously presents a cancer stem cell antigen. Therefore, it was shown that the antibody of the present disclosure is capable of injuring a cell which endogenously presents a natural antigenic peptide.

Moreover, Caki-1 cell was co-cultured for 7 hours with either Clone A10 or Clone B10 in serum-containing medium at 37 °C under 5% CO₂ condition, then analyzed by FACS. Both red fluorescence from PE and blue fluorescence from DAPI were observed in 4.23 % cells for Clone A10 and 23.36 % cells for Clone B10.

### Example 5. Analysis of bispecific antibody BiTE

### (1) Generation of BiTEs and affinities to various cells

A bispecific antibody BiTE was generated for each of Clone A10, Clone B10, Clone 4 and Clone 11, by linking the scFv of each of the antibodies and the scFv of an anti-CD3 (SEQ ID NO: 21), using a similar method to that described in Stadler et al., Nature Medicine volume 23, pages 815-817 (2017). Such BiTEs were used to examine their affinities to a cancer stem cell antigenic peptide-pulsed T2-A24 cell, T cell and NK cell. Each BiTE exhibited an affinity to the cancer stem cell antigenic peptide-pulsed T2-A24 cell. It also exhibited a high affinity to the T cell, but it exhibited no affinity to the NK cell.

### (2) Cytotoxic activity

The BiTE generated in (1) was mixed with T cell, and co-cultured with the cancer stem cell antigenic peptide-pulsed T2-A24 cell in the presence of FITC-labelled annexin V and propidium iodide (PI). The fluorescence was analyzed after 2.5 hours and 5 hours by FACS. In the case of the controls (with no treatment or with only T cell), there was little change in cells in which both FITC and PI fluorescence were observed between after 2.5 hours and after 5 hours, whereas in the case of co-culturing in the presence of a BiTE, a remarkable increase was indicated in cells in which both FITC and PI fluorescence were observed after 5 hours as compared to that after 2.5 hours.

### Example 6. Screening for HLA-A02/natural antigenic peptide complex-specific antibodies

### (1) Generation of antibody-phages

A natural antigenic peptide LV9 (SEQ ID NO: 23) derived from a polypeptide expressed by SEQ ID NO: 22, a unique sequence to BORIS sf6, was used as a cancer stem cell antigenic peptide to identify the scFvs that recognize HLA-A02/LV9 complex using a similar method to the phage-display method described in Tsukahara et al., J Biol Chem., 2014, Aug 8;289(32):22035-47. In Example 6, the "cancer stem cell antigenic peptide" means LV9.

Specifically, first, from the scFv library, a phagemid vector was generated in which a DNA encoding for a peptide in which the heavy-chain variable region (VH region) and light-chain variable region (VL region) of an antibody were connected by a linker has been integrated, used to infect *Escherichia coli.* This was further infected with a helper phage to generate an M13 phage presenting an scFv in which the VH region and the VL region were connected with a linker (the antibody phage).

### (2) Biopanning

Prior to the biopanning, the nonspecifically-bound phages were removed from the phage library using a biotin-modified complex of HLA-A02 and HIV peptide (SLYNTVATL (SEQ ID NO: 39)) and streptavidin-bound magnetic beads. From the remaining antibody phages, using the biotin-modified HLA-A02/LV9 complex and streptavidin-bound magnetic beads, the antibody phages which bind to each complex were screened (positive panning). This step was repeated three times to give candidate antibody phages which specifically bind to the HLA-A02/natural antigenic peptide complex.

### (3) Conversion of scFv antibody to IgG1-type and FACS analysis

The scFv part was separated from the obtained candidate antibody phage, labelled with a FLAG tag at the end of the VL region, and the reactivity to HLA-A02/LV9 complex was analyzed using FACS. As a result of the analysis, from 23 candidate scFvs, clones with high reactivity were screened, the heavy chain Fc region of human IgG1 (CH2 and CH3 regions, SEQ ID NO: 19) was linked instead of FLAG tag, and the scFv antibodies were converted to IgG1-type. The antibodies converted to IgG1-type (scFv-hIgG1) were used to carry out further FACS analysis, and the clones with particularly high reactivity (LV9: Clone 11, Clone 13 and Clone 19) were screened.

For all antibodies, the reactivity was high at the concentration of 200 µg/mL, though that of Clone 19 was the highest. As negative controls, cells which were pulsed with HIV (SLYNTVATL (SEQ ID NO: 39)), EBV (YLQQNWWTL (SEQ ID NO: 40)), or CMV (NLVPMVATV (SEQ ID NO: 41)) peptide at 200 µg/mL each were used. The results are shown in Fig. 7.

The sequences of the CDR1 to CDR3 regions of these clones were as follows:
Clone 11 VH region
   Sequence of CDR1 region: GFTFDKYG (SEQ ID NO: 24)
   Sequence of CDR2 region: ITWNSGKV (SEQ ID NO: 25)
   Sequence of CDR3 region: CARDLGYYYDSSGYLKPTGSGFDYW (SEQ ID NO: 26)
Clone 11 VL region
   Sequence of CDR1 region: SSNIGAGYD (SEQ ID NO: 27)
   Sequence of CDR2 region: GNT
   Sequence of CDR3 region: CGTWDSSLSVVLF (SEQ ID NO: 28)
Clone 13 VH region
   Sequence of CDR1 region: GYTFTGYY (SEQ ID NO: 29)
   Sequence of CDR2 region: ISTVFNTP (SEQ ID NO: 30)
   Sequence of CDR3 region: CARSYSGSLQDAFDIW (SEQ ID NO: 31)
Clone 13 VL region
   Sequence of CDR1 region: NLKRKN (SEQ ID NO: 32)
   Sequence of CDR2 region: DGT
   Sequence of CDR3 region: CQVWDIDSEDYVF (SEQ ID NO: 33)
Clone 19 VH region
   Sequence of CDR1 region: TYSFSNYW (SEQ ID NO: 34)
   Sequence of CDR2 region: IYPGDPDT (SEQ ID NO: 35)
   Sequence of CDR3 region: CARRWAARPDDAFDIW (SEQ ID NO: 36)
Clone 19 VL region
   Sequence of CDR1 region: NIESKI (SEQ ID NO: 37)
   Sequence of CDR2 region: HDS
   Sequence of CDR3 region: CQVWDSSSDHVVF (SEQ ID NO: 38)

### [Industrial Applicability]

The antibodies of the present disclosure are capable of reacting to a endogenously presented natural antigenic peptide, and further exhibit a cytotoxic activity through humoral immunity reaction. This enables an immunotherapy that is targeted to cancer stem cells to be carried out for a patient with an intractable cancer and sarcoma which exhibits resistance against existing therapies. Moreover, because its cytotoxic mechanism is exerted via humoral immunity, it is possible to perform a cancer vaccine immunotherapy which is not dependent on the immunity of the subject to whom it is administered. Accordingly, it can be expected to exhibit a high effect both as an anti-cancer agent and as a cancer immunotherapy.

## Claims

1. A multispecific antibody having an antigen-binding site that recognizes a complex of an antigenic peptide derived from a protein selected from a group consisting of DNAJB8 protein, BORIS sf6 and FAM83B protein and an MHC molecule.

2. The multispecific antibody according to Claim 1, further having an antigen-binding site that recognizes CD3.

3. The multispecific antibody according to Claim 1 or 2, wherein the antigenic peptide is a peptide consisting of an amino acid sequence set forth in SEQ ID NOs: 5, 23 or 6.

4. The multispecific antibody according to any one of Claims 1-3, wherein the antigen-binding site that recognizes the complex of an antigenic peptide derived from a protein selected from a group consisting of DNAJB8 protein, BORIS sf6 protein and FAM83B protein and an MHC molecule comprises an amino acid sequence set forth in any one of SEQ ID NOs: 7-14 and 24-38, or an amino acid sequence set forth in any one of the amino acid SEQ ID NOs: 7-14 and SEQ ID NOs: 24-38 in which one or two amino acid(s) are substituted.

5. The multispecific antibody according to any one of Claims 1-4, further comprising a CD80 region.

6. A pharmaceutical composition comprising an antibody that recognizes a complex of an antigenic peptide derived from a protein selected from a group consisting of DNAJB8 protein, BORIS sf6 protein and FAM83B protein and an MHC molecule.

7. The pharmaceutical composition according to Claim 6, for preventing and/or treating cancer.

8. The pharmaceutical composition according to Claim 6 or 7, wherein the MHC molecule is an HLA molecule.

9. The pharmaceutical composition according to any one of Claims 6-8, wherein the antigenic peptide consists of an amino acid sequence set forth in SEQ ID NOs: 5, 23 or 6.

10. The pharmaceutical composition according to any one of Claims 6-9, wherein the antigen-binding site of the antibody comprises an amino acid sequence set forth in any one of SEQ ID NOs: 7-14 and 24-38.

11. The pharmaceutical composition according to any one of Claims 6-10, wherein the antibody is the multispecific antibody according to any one of Claims 1-5.


### [Sequence Listing]

SEQUENCE LISTING

<110>  Sapporo Medical University

<120>  Cancer Stem Cell Specific Antibody

<130>  PCT3001SI

<150>  JP2018189834
<151>  2018-10-05

<160>  41

<170>  PatentIn version 3.5

<210>  1
<211>  2475
<212>  DNA
<213>  Homo sapiens

<400>  1
acaccgcctt tgccatcacc accgtcattg ccccctctac taccaccacc atcaccacca      60

ccgggtctgc ctcagccaga cccaccactg cctctaatta gactgccacc atcatcactg     120

ccaccaccac tgcccctcca atgccttcct gatgtcacca tctgctttct ctctgctatc     180

ctcacaccca actccatcac aaccaccacc ttcatctgag ccgccatcac cctcacagta     240

cacagccctc aaatgtgtac caaggaggag gacatttact acacgctgca ccctgaacag     300

tctgggacca gcggtcaggg aacaaggaat cgagatgctc acctgcagct cccaggtgag     360

cggctctcta gagcttgcct gggagctgct gaggagctca cggtattcca ggaagctccc     420

catccatgcc tcagcctgtg gctcagaagc agggtcttcg cagaagattg ccccgagctg     480

ttgcaaagct caccattgtc acctgcctgc aacggcctct cttttccact ctccaaatcc     540

cttgtattgg caggctctag tctggaatta taccggtgat gggattctgg aaaatccctc     600

agatttctcc aggatgcaag gagaccatgg aagaggaaca agcctgcaag ggacagcagg     660

tgctaactgc taaccgagac atagtgacaa aaatccagcc acagagataa agaatcaggt     720

tcatctgtag ctacatccag tggagaagtt ctgctcttca actcctctgc taagccacat     780

gtgtcagcag gtgtagaatt gagtggaaca cttctttgga tttgctgtcc gctgcagcct     840

tggccctggt ttcacatccc ctccctcact gactcgctgt gtgaccttga gcactgtgtg     900

tctctgagcc tggttcatcc aagagtttac cgacggccct cgtgtgccag ccactgtcct     960

agacagggac agactttccc gcctttgtgg agcttacccc ggaagacctg gccagataat    1020

gggcacaaaa aaagcaggct cttgtcccca ccccagcctg cctgaacccc accttggcct    1080

cctcttgcct cagctgctgc ccagccatcg gccgagtgga catacccagg acgcccttgc    1140

ccgcctcccc caccacggcc acagaccttt gtcactcacc aaggctggaa tcccgtggct    1200

gatcctgcct ctgccctttg gctcccggga ctcagcccac accacctggg tcacagagca    1260

tcccattccc acacaccgtt gtggccacct caccagcagg ggcaggccca tatgcccagg 1320

tttgcctggt gaggagctgg gggcgggggt atgccccgcc ccgggagctg acgtcataaa 1380

aggagctctg gagggcagcc cactctggcc tggccccaca gcggcagtgt ccctcccctc 1440

cccccactcc tctcagtggg ggcccctcca gtccctgaga attggtacta cgaaaaggtg 1500

aactcctggg cagaatcttg cctagagctt gcggagtcca gccaggcccc tgctgaaggg 1560

ccccagacca ccggccactt ctcccccgtc catctgacca gctgggcccc tgcgcccacc 1620

tggcctccac gttccctctc ctctcaccca cacccctggc catggctaac tactacgaag 1680

tgctgggcgt gcaggccagc gcttccccgg aggacatcaa gaaagcctac cgcaagctgg 1740

cccttcgttg gcaccccgac aagaaccctg acaataagga ggaggcggag aagaagttca 1800

agctggtgtc tgaggcctat gaggttctgt ctgactccaa gaaacgctcc ctgtatgacc 1860

gtgctggctg tgacagctgg cgggctggtg gcggggccag cacgccctac cacagcccct 1920

tcgacaccgg ctacaccttc cgtaaccctg aggacatctt ccgggagttt tttggtggcc 1980

tggacccttt ctcctttgag ttctgggaca gcccattcaa tagtgaccgt ggtggccggg 2040

gccatggcct gaggggggcc ttctcggcag gctttggaga atttccggcc ttcatggagg 2100

ccttctcatc cttcaacatg ctgggctgca gcgggggcag ccacaccacc ttctcatcca 2160

cctccttcgg gggctccagt tctggcagct cggggttcaa gtcggtgatg tcgtccaccg 2220

agatgatcaa tggccacaag gtcaccacca agcgcatcgt ggagaacggg caggagcgcg 2280

tggaggtgga ggaagacggg cagctcaagt cggtgactgt gaacggcaag gagcagctca 2340

aatggatgga cagcaagtag gcgctggcca cccggccctg ccttcccacc accaccaccg 2400

tgcatggggc agcaaacacg tggggccgca gacatagcct gatggttaat aaatgtgcca 2460

agtgagttca tggca 2475

<210> 2
<211> 232
<212> PRT
<213> Homo sapiens

<400> 2

Met Ala Asn Tyr Tyr Glu Val Leu Gly Val Gln Ala Ser Ala Ser Pro
1               5                   10                  15

Glu Asp Ile Lys Lys Ala Tyr Arg Lys Leu Ala Leu Arg Trp His Pro
            20                  25                  30

Asp Lys Asn Pro Asp Asn Lys Glu Glu Ala Glu Lys Lys Phe Lys Leu
        35                  40                  45

Val Ser Glu Ala Tyr Glu Val Leu Ser Asp Ser Lys Lys Arg Ser Leu

50                  55                  60

Tyr Asp Arg Ala Gly Cys Asp Ser Trp Arg Ala Gly Gly Gly Ala Ser
65                  70                  75                  80

Thr Pro Tyr His Ser Pro Phe Asp Thr Gly Tyr Thr Phe Arg Asn Pro
                85                  90                  95

Glu Asp Ile Phe Arg Glu Phe Phe Gly Gly Leu Asp Pro Phe Ser Phe
            100                 105                 110

Glu Phe Trp Asp Ser Pro Phe Asn Ser Asp Arg Gly Gly Arg Gly His
        115                 120                 125

Gly Leu Arg Gly Ala Phe Ser Ala Gly Phe Gly Glu Phe Pro Ala Phe
    130                 135                 140

Met Glu Ala Phe Ser Ser Phe Asn Met Leu Gly Cys Ser Gly Gly Ser
145                 150                 155                 160

His Thr Thr Phe Ser Ser Thr Ser Phe Gly Gly Ser Ser Ser Gly Ser
                165                 170                 175

Ser Gly Phe Lys Ser Val Met Ser Ser Thr Glu Met Ile Asn Gly His
            180                 185                 190

Lys Val Thr Thr Lys Arg Ile Val Glu Asn Gly Gln Glu Arg Val Glu
        195                 200                 205

Val Glu Glu Asp Gly Gln Leu Lys Ser Val Thr Val Asn Gly Lys Glu
    210                 215                 220

Gln Leu Lys Trp Met Asp Ser Lys
225                 230

<210>  3
<211>  6269
<212>  DNA
<213>  Homo sapiens

<400>  3
aaaccttcgg cggccggcgc tgtgcggcgg gcgcggttgc gcgcggcttg gggcaaatac      60

ttctcaccac tgcatgaatg gacatttgaa agtgccatag ccaaacactt gcaagcatgg     120

agacctcatc aatgctttcc tcattgaatg atgagtgtaa atctgacaac tacattgagc     180

ctcactacaa ggaatggtat cgagtagcca ttgatattct gattgaacac gggttagaag     240

cataccaaga atttcttgtc caggaacgag tttcagactt tcttgctgag gaagaaatta     300

attatatttt gaaaaatgtc cagaaagttg cacaaagcac agcacatggt actgatgatt 360

cctgtgatga taccttatct tcagggacct actggcctgt tgagtctgat gtggaagctc 420

caaatcttga cttaggctgg ccatatgtga tgcccggact cttagggggc acccatatag 480

atctcctttt tcatccacca agagcacatc tacttacgat aaaagaaact attcggaaga 540

tgataaaaga agcaagaaag gtcattgctt tagtgatgga tatatttaca gatgtggaca 600

ttttcaaaga aatcgttgag gcatcaactc gaggagtatc tgtttacatt ctgcttgatg 660

agtccaattt taatcatttt ctaaatatga ctgagaaaca aggttgttca gttcagcgtc 720

tcaggaatat tcgagtgcga acagtaaaag gccaagatta tctttcaaaa acaggggcaa 780

aattccatgg aaaaatggaa cagaaatttt tgttagttga ctgccagaaa gtgatgtacg 840

gttcttacag ttatatgtgg tcatttgaga aagctcacct cagcatggtt cagataatta 900

caggacaact tgttgagtcc tttgatgaag aatttagaac tctctatgcc agatcctgtg 960

tccctagttc atttgctcag gaagaatcag caagggtgaa gcatggaaaa gccctctggg 1020

aaaatggcac ttaccagcat tcggtgtctt cattagcatc tgtttccagc cagagaaacc 1080

tttttggtag acaagacaag attcataaac tagattccag ttacttcaaa aacagaggga 1140

tatatacttt aaatgaacat gacaaatata acataagaag tcacggatac aaacctcatt 1200

ttgttcctaa ctttaatggt ccaaacgcaa tacgtcagtt tcaacccaat cagataaatg 1260

aaaattggaa aaggcatagt tatgctgggg aacagccaga aacagtgcca tacctcctgc 1320

ttaatagggc tctgaataga accaataatc cacctggtaa ttggaaaaag ccatctgata 1380

gtctcagtgt ggcgtcctca tcacgggaag gctatgtaag ccaccacaac acacctgccc 1440

agagttttgc caatcggctt gcgcagagaa aaacaacaaa tcttgcagac aggaattcaa 1500

atgttcggag gtcttttaat gggacagata accatatccg ctttttgcaa caacgaatgc 1560

caacccttga acataccaca aagtcattcc tacgtaactg gagaattgaa tcctacttaa 1620

atgatcattc agaagctaca ccggactcaa atggatcagc tttaggtgac cgatttgagg 1680

gctatgataa tcctgagaat ttgaaggcca atgcccttta tactcattct cggcttcgtt 1740

cctctttagt atttaaaccc actttacctg agcaaaagga agttaacagt tgtacaactg 1800

gctcctcaaa ttcaactatc attggttctc agggaagtga gacacctaaa gaggtcccag 1860

acacccctac gaatgtacag catttgacag acaaaccctt gccagaatca atccccaagc 1920

tcccattgca gtcagaggca ccaaaaatgc acaccttgca ggttcctgaa aaccactcag 1980

tagccttaaa ccaaactaca aatggccata ctgaatcaaa taactatata tataaaacct 2040

tgggtgtaaa taagcagaca gaaaatctaa agaatcaaca gactgagaat ctacttaaaa 2100

ggcgaagttt cccgttattt gacaactcaa aagccaactt agatcctgga aatagtaagc 2160

attatgtata tagtacactt accaggaatc gagttagaca accagaaaag cccaaagaag 2220

atttgctgaa aagttctaaa agcatgcaca atgtgactca taacttggag gaggatgagg 2280

aggaagttac caagagaaac tctccaagtg gcactactac caaatcagtt tccattgctg 2340

ctttacttga tgtgaataaa gaggaatcta acaaagaact tgcttcaaag aaggaagtta 2400

agggttcccc aagttttttg aaaaaggggt ctcagaagtt aaggtcatta cttagcctta 2460

ccccagataa gaaagaaaat ctatccaaaa ataaagcacc tgccttttat agattgtgta 2520

gtagctctga cacattagtt tctgagggtg aagaaaatca aaaaccaaag aaatcagaca 2580

caaaagttga ttcatctcct agaagaaagc attcttcctc atcgaattct caaggcagca 2640

tccacaagag taaggaagat gtaacagtta gcccatctca agagataaat gctccaccag 2700

atgaaaataa aagaacacct tctccaggtc cagttgaaag caagttcttg gaaagggcag 2760

gagatgcctc tgccccaaga tttaacactg aacagatcca ataccgagat tcaagggaga 2820

ttaatgcagt tgttacccct gaaagaagac ctacttcttc tccaaggcca acgtccagtg 2880

agcttctacg atctcattca actgatcggc gtgtttacag tcgttttgag ccgttttgta 2940

agattgagag ctctattcag ccaacaagca acatgccaaa taccagtata aatcgcccag 3000

aaataaaatc tgcgactatg ggcaacagtt atggcaggtc tagtccattg cttaattaca 3060

acactggtgt ttatcgctca tatcaaccca atgagaacaa gtttcgagga tttatgcaaa 3120

agtttggaaa ctttatacac aaaaataaat agctattaaa atgcaaaatg aatgaggcta 3180

tcaatatttg tccaaagaaa attgtggaca gtctttgtaa catgccaata gatttcccta 3240

aggacagaat tatgggtatg atgtatatgt tcaccagtgt cctagtataa agttattttt 3300

ctgtgtgata aagttagggt ctgctgtaga tagaatttct ctgtaaacac attatttgta 3360

agtggtaatg gtaaaaataa tagatgtatt taaatcattt tctttagact gaagatttta 3420

agtctcactt agaaattttt gtggacgatg tatggtgtat ggtgtatggt gtatggattt 3480

taaccatttc cttttaaaaa ggtcatacta ccctcagtaa ccctttataa catgtgttta 3540

tagtgttttc atatcttcaa aaatataagc aaatgggaca aagccctttt ttaagattaa 3600

cttgaagttc tacgggataa cttgttatat tttattaata ttattttttc tgtggatcat 3660

tgtacagctg tttgggcaac agcagtacct tttacatttt ttatttttta tttttttttc 3720

tgcctgaagt gtttgcaaag tatcagcctc atgtagtata atgacattcg taggaattta 3780

ttttcctcag cctttgaaaa cggtctggga tgtgcttctt tctacactgg acccagggag 3840

ctgtccccct cttacccata ggctgctgat tttttatagt cattccttac ttcacattta 3900

ggacaaacca gtaggaattt agaaaatctg aatgattccc cctccttttt ctattgtata 3960

aaagctttta gaaatgtaaa tttctgccta aatttttgtt agattggcat gacaatgcta 4020

aggggtcttg cttgcgaaaa ttcttgctct tttttttttt cttttgagat ggagtctcgc 4080

tctgtcaccc aggctagagt gcagtggcgc aatcttggct cactgcaacc tctgcctccc 4140

aagttcaaac gattctcctg cctcagcctc ctgagtagct gggattacag gtgcgcacca 4200

ccacacccgg ctaatttttg tattttcaga agagatggga tttcaccatg ttggccagtc 4260

tagtctcaaa ctcctgacct cgtgatcctc ccgcctcggc ctcccagagt gctgggatta 4320

caggtcttgc tcttctttaa gttgtgccaa atatgatatg actgcatagt gtttttgtaa 4380

gaataccatt gggaaaatga gaagttttac tgggctagtt cttccatgat ttgaggatga 4440

tattcagatt ataagatacc ctttgtcttt cttacagtaa tgaagtataa gctccatatc 4500

tgttccagag actttaactt gactttgctt cattcacaaa gaacagagtt caagaagcag 4560

tgcatcctgt gagaagtgtg aagtgtttgt acatcacttt aaatatatta cttaatatat 4620

tctaagttgc tgtgtggagc agtatactgt tgttttaaaa atgcaagatt cagacaaatt 4680

ttaatattgt ctcattaaaa taatttaaat aatggtataa aatatctata tcattaaaat 4740

aattttccat agtgtttaga aaccatgaaa aagaaaacat agcaggagaa aatatgacag 4800

gaaaagaaaa cctaacaaag cccagaaccc acagttaacc aaactagact gactttgtta 4860

ttacccattc tttgttagta ttggataggg aattaggata atgagccatt aggcagttct 4920

gaaatggaaa gccctgaaat ttagtgcaat gtaactttaa aactgaaatt atataaagag 4980

tagaagttta atcatgttta attacaacca aaagcctgtt gcctttttgt acagaaatct 5040

ccttaatttc aggacatgta gatagcttat agaaacatcc ttttaaatag tttgtgagct 5100

cttcctcttc agtggaattg aggaatacag aatgctttat ttcatcatcc cctgacaggt 5160

gacttaggct ttgcacagca gatttatttt tctctgcgtt ttttaaaaat tgtttttctt 5220

gtatcttttt tcttcaaaaa tatctatatt tgagaatatg tacataacaa ctttccaaag 5280

tctctgtggc caaaacctct ccagggataa gactgagcaa gaatataata cttcaaaaaa 5340

tgtacagcta ctgtttaagt tttaaacaga caccatcaca gtttgtggat gaaatagttt 5400

taagccatat actttctgtc tttttttccc catattaata ttggggggcg gataatatca 5460

ctttgatgta cattgatatt aaagtttggt aatgcagctt ttactgtcta catggtactg 5520

tacattagtt tttaagcaga aacacaagaa aaatgggtat aatttcaaag tagttcttgg 5580

cagatggcta gagaatactg caagtgaccc tgtatcccga atacacagat atccctctat 5640

tacaagtttg ggattagcca taattctgac atggtgtgtt caggtatggg tatatgttgt 5700

cagtctacac ttgtggaagc aaatatcttg tttaatcaag atgatgtcta gtgtcaccta 5760

aataatgcaa aaagtttaat tctggatgaa ttcagcttta ctcaagatcc acatattcaa 5820

gtatcattcc acagatattc acagaacaca gaaatatctg tgttctgcct tatgcctgtg 5880

gctaagggat gcaaagtaga attgctttac attgactata tatgtgacat gtacgttgct 5940

gtttttttta aaataacttt atcatgatat tcaggtagat cctgggttct agaatattta 6000

aaacaaaagg ataaaatgat aaaccaaaga gtcaacttgt taacttttct tttttaagag 6060

atgggttttc actagtatgc ccagtctgga ctccaagtcc tgggctcaaa cgatcctcca 6120

gcctcaggtt cctgagtagc tcaacatttt atgtatgtac gatattataa agaaatattc 6180

ttccaaatga acttttgttt ttagatcaat aaatgaataa taaataattt tgtacaaaca 6240

tcaaaaaaaa aaaaaaaaaa aaaaaaaaa 6269

<210> 4
<211> 1011
<212> PRT
<213> Homo sapiens

<400> 4

Met Glu Thr Ser Ser Met Leu Ser Ser Leu Asn Asp Glu Cys Lys Ser
1               5                   10                  15

Asp Asn Tyr Ile Glu Pro His Tyr Lys Glu Trp Tyr Arg Val Ala Ile
            20                  25                  30

Asp Ile Leu Ile Glu His Gly Leu Glu Ala Tyr Gln Glu Phe Leu Val
        35                  40                  45

Gln Glu Arg Val Ser Asp Phe Leu Ala Glu Glu Glu Ile Asn Tyr Ile
    50                  55                  60

Leu Lys Asn Val Gln Lys Val Ala Gln Ser Thr Ala His Gly Thr Asp
65                  70                  75                  80

Asp Ser Cys Asp Asp Thr Leu Ser Ser Gly Thr Tyr Trp Pro Val Glu
                85                  90                  95

Ser Asp Val Glu Ala Pro Asn Leu Asp Leu Gly Trp Pro Tyr Val Met
            100                 105                 110

Pro Gly Leu Leu Gly Gly Thr His Ile Asp Leu Leu Phe His Pro Pro
        115                 120                 125

Arg Ala His Leu Leu Thr Ile Lys Glu Thr Ile Arg Lys Met Ile Lys
    130                 135                 140

Glu Ala Arg Lys Val Ile Ala Leu Val Met Asp Ile Phe Thr Asp Val
145                 150                 155                 160

Asp Ile Phe Lys Glu Ile Val Glu Ala Ser Thr Arg Gly Val Ser Val
                165                 170                 175

Tyr Ile Leu Leu Asp Glu Ser Asn Phe Asn His Phe Leu Asn Met Thr
        180                 185                 190

Glu Lys Gln Gly Cys Ser Val Gln Arg Leu Arg Asn Ile Arg Val Arg
    195                 200                 205

Thr Val Lys Gly Gln Asp Tyr Leu Ser Lys Thr Gly Ala Lys Phe His
    210                 215                 220

Gly Lys Met Glu Gln Lys Phe Leu Leu Val Asp Cys Gln Lys Val Met
225                 230                 235                 240

Tyr Gly Ser Tyr Ser Tyr Met Trp Ser Phe Glu Lys Ala His Leu Ser
                245                 250                 255

Met Val Gln Ile Ile Thr Gly Gln Leu Val Glu Ser Phe Asp Glu Glu
            260                 265                 270

Phe Arg Thr Leu Tyr Ala Arg Ser Cys Val Pro Ser Ser Phe Ala Gln
        275                 280                 285

Glu Glu Ser Ala Arg Val Lys His Gly Lys Ala Leu Trp Glu Asn Gly
    290                 295                 300

Thr Tyr Gln His Ser Val Ser Ser Leu Ala Ser Val Ser Ser Gln Arg
305                 310                 315                 320

Asn Leu Phe Gly Arg Gln Asp Lys Ile His Lys Leu Asp Ser Ser Tyr
                325                 330                 335

Phe Lys Asn Arg Gly Ile Tyr Thr Leu Asn Glu His Asp Lys Tyr Asn
            340                 345                 350

Ile Arg Ser His Gly Tyr Lys Pro His Phe Val Pro Asn Phe Asn Gly
        355                 360                 365

Pro Asn Ala Ile Arg Gln Phe Gln Pro Asn Gln Ile Asn Glu Asn Trp
    370                 375                 380

Lys Arg His Ser Tyr Ala Gly Glu Gln Pro Glu Thr Val Pro Tyr Leu
385                 390                 395                 400

Leu Leu Asn Arg Ala Leu Asn Arg Thr Asn Asn Pro Pro Gly Asn Trp
                405                 410                 415

Lys Lys Pro Ser Asp Ser Leu Ser Val Ala Ser Ser Ser Arg Glu Gly
            420                 425                 430

Tyr Val Ser His His Asn Thr Pro Ala Gln Ser Phe Ala Asn Arg Leu
        435                 440                 445

Ala Gln Arg Lys Thr Thr Asn Leu Ala Asp Arg Asn Ser Asn Val Arg
    450                 455                 460

Arg Ser Phe Asn Gly Thr Asp Asn His Ile Arg Phe Leu Gln Gln Arg
465                 470                 475                 480

Met Pro Thr Leu Glu His Thr Thr Lys Ser Phe Leu Arg Asn Trp Arg
                485                 490                 495

Ile Glu Ser Tyr Leu Asn Asp His Ser Glu Ala Thr Pro Asp Ser Asn
            500                 505                 510

Gly Ser Ala Leu Gly Asp Arg Phe Glu Gly Tyr Asp Asn Pro Glu Asn
        515                 520                 525

Leu Lys Ala Asn Ala Leu Tyr Thr His Ser Arg Leu Arg Ser Ser Leu
    530                 535                 540

Val Phe Lys Pro Thr Leu Pro Glu Gln Lys Glu Val Asn Ser Cys Thr
545                 550                 555                 560

Thr Gly Ser Ser Asn Ser Thr Ile Ile Gly Ser Gln Gly Ser Glu Thr
                565                 570                 575

Pro Lys Glu Val Pro Asp Thr Pro Thr Asn Val Gln His Leu Thr Asp
            580                 585                 590

Lys Pro Leu Pro Glu Ser Ile Pro Lys Leu Pro Leu Gln Ser Glu Ala
        595                 600                 605

Pro Lys Met His Thr Leu Gln Val Pro Glu Asn His Ser Val Ala Leu
    610                 615                 620

Asn Gln Thr Thr Asn Gly His Thr Glu Ser Asn Asn Tyr Ile Tyr Lys
625                 630                 635                 640

Thr Leu Gly Val Asn Lys Gln Thr Glu Asn Leu Lys Asn Gln Gln Thr
                645                 650                 655

Glu Asn Leu Leu Lys Arg Arg Ser Phe Pro Leu Phe Asp Asn Ser Lys
            660                 665                 670

Ala Asn Leu Asp Pro Gly Asn Ser Lys His Tyr Val Tyr Ser Thr Leu
        675                 680                 685

Thr Arg Asn Arg Val Arg Gln Pro Glu Lys Pro Lys Glu Asp Leu Leu
    690                 695                 700

Lys Ser Ser Lys Ser Met His Asn Val Thr His Asn Leu Glu Glu Asp
705                 710                 715                 720

Glu Glu Glu Val Thr Lys Arg Asn Ser Pro Ser Gly Thr Thr Thr Lys
                725                 730                 735

Ser Val Ser Ile Ala Ala Leu Leu Asp Val Asn Lys Glu Glu Ser Asn
            740                 745                 750

Lys Glu Leu Ala Ser Lys Lys Glu Val Lys Gly Ser Pro Ser Phe Leu
        755                 760                 765

Lys Lys Gly Ser Gln Lys Leu Arg Ser Leu Leu Ser Leu Thr Pro Asp
    770                 775                 780

Lys Lys Glu Asn Leu Ser Lys Asn Lys Ala Pro Ala Phe Tyr Arg Leu
785                 790                 795                 800

Cys Ser Ser Ser Asp Thr Leu Val Ser Glu Gly Glu Glu Asn Gln Lys
                805                 810                 815

Pro Lys Lys Ser Asp Thr Lys Val Asp Ser Ser Pro Arg Arg Lys His
            820                 825                 830

Ser Ser Ser Ser Asn Ser Gln Gly Ser Ile His Lys Ser Lys Glu Asp
        835                 840                 845

Val Thr Val Ser Pro Ser Gln Glu Ile Asn Ala Pro Pro Asp Glu Asn
    850                 855                 860

Lys Arg Thr Pro Ser Pro Gly Pro Val Glu Ser Lys Phe Leu Glu Arg
865                 870                 875                 880

Ala Gly Asp Ala Ser Ala Pro Arg Phe Asn Thr Glu Gln Ile Gln Tyr
                885                 890                 895

Arg Asp Ser Arg Glu Ile Asn Ala Val Val Thr Pro Glu Arg Arg Pro
            900                 905                 910

Thr Ser Ser Pro Arg Pro Thr Ser Ser Glu Leu Leu Arg Ser His Ser
        915                 920                 925

Thr Asp Arg Arg Val Tyr Ser Arg Phe Glu Pro Phe Cys Lys Ile Glu

930                 935                 940

Ser Ser Ile Gln Pro Thr Ser Asn Met Pro Asn Thr Ser Ile Asn Arg
945                 950                 955                 960

Pro Glu Ile Lys Ser Ala Thr Met Gly Asn Ser Tyr Gly Arg Ser Ser
                965                 970                 975

Pro Leu Leu Asn Tyr Asn Thr Gly Val Tyr Arg Ser Tyr Gln Pro Asn
            980                 985                 990

Glu Asn Lys Phe Arg Gly Phe Met  Gln Lys Phe Gly Asn  Phe Ile His
        995                 1000                1005

Lys Asn  Lys
    1010

<210>  5
<211>  9
<212>  PRT
<213>  Artificial Sequence

<220>
<223>  natural antigen peptide derived from DNAJB8 protein

<400>  5

Ala Phe Met Glu Ala Phe Ser Ser Phe
1               5

<210>  6
<211>  9
<212>  PRT
<213>  Artificial Sequence

<220>
<223>  natural antigen peptide derived from FAM83B

<400>  6

Ser Tyr Gln Pro Asn Glu Asn Lys Phe
1               5

<210>  7
<211>  16
<212>  PRT
<213>  Artificial Sequence

<220>
<223>  heavy chain CDR3 region of clone A10

<400>  7

Cys Ala Arg Val Gly Trp Asp Leu Leu Gly Arg Ala Phe Asp Ile Trp
1               5                   10                  15

<210>  8
<211>  13
<212>  PRT
<213>  Artificial Sequence

<220>
<223>  light chain CDR3 region of clone A10

<400>  8


<210>  9
<211>  18
<212>  PRT
<213>  Artificial Sequence

<220>
<223>  heavy chain CDR3 region of clone B10

<400>  9

Cys Ala Arg Asp Gly Glu Met Ala Thr Val Val Ala Gly Pro Phe Asp
1               5                   10                  15


Asn Trp



<210>  10
<211>  10
<212>  PRT
<213>  Artificial Sequence

<220>
<223>  light chain CDR3 region of clone B10

<400>  10


<210>  11
<211>  15
<212>  PRT
<213>  Artificial Sequence

<220>
<223>  heavy chain CDR3 region of clone 4

<400>  11

Cys Ala Lys Asp Ile Gly Ser Gly Trp Phe Ser Met Asp Val Trp
1               5                   10                  15


<210>  12

<211>  15
<212>  PRT
<213>  Artificial Sequence

<220>
<223>  light chain CDR3 region of clone 4

<400>  12

Cys Asn Ser Arg Asp Tyr Ser Gly Asn His Arg Gly Val Leu Phe
1               5                   10                  15


<210>  13
<211>  16
<212>  PRT
<213>  Artificial Sequence

<220>
<223>  heavy chain CDR3 region of clone 10

<400>  13

Cys Ala Lys Gly Lys Tyr Ser Gly Ser Tyr Tyr Ala Leu Asp Tyr Trp
1               5                   10                  15


<210>  14
<211>  13
<212>  PRT
<213>  Artificial Sequence

<220>
<223>  light chain CDR3 region of clone 10

<400>  14


<210>  15
<211>  11
<212>  PRT
<213>  Artificial Sequence

<220>
<223>  HLA-A24 restricted peptide derived from HIV

<400>  15

Arg Tyr Leu Arg Asp Gln Gln Leu Leu Gly Ile
1               5                   10


<210>  16
<211>  9
<212>  PRT
<213>  Artificial Sequence

<220>
<223>  HLA-A24 restricted peptide derived from EBV

<400>  16

Thr Tyr Gly Pro Val Phe Met Ser Leu
1               5


<210>  17
<211>  9
<212>  PRT
<213>  Artificial Sequence

<220>
<223>  HLA-A24 restricted peptide derived from CMV

<400>  17

Gln Tyr Asp Pro Val Ala Ala Leu Phe
1               5


<210>  18
<211>  684
<212>  DNA
<213>  Artificial Sequence

<220>
<223>  hIgG1 CH2/CH3

<400>  18
gacaaaactc acacatgccc accgtgccca gcacctgaac tcctgggggg accgtcagtc      60

ttcctcttcc ccccaaaacc caaggacacc ctcatgatct cccggacccc tgaggtcaca     120

tgcgtggtgg tggacgtgag ccacgaagac cctgaggtca agttcaactg gtacgtggac     180

ggcgtggagg tgcataatgc caagacaaag ccgcgggagg agcagtacaa cagcacgtac     240

cgtgtggtca gcgtcctcac cgtcctgcac caggactggc tgaatggcaa ggagtacaag     300

tgcaaggtct ccaacaaagc cctcccagcc cccatcgaga aaaccatctc caaagccaaa     360

gggcagcccc gagaaccaca ggtgtacacc ctgcccccat cccgggagga gatgaccaag     420

aaccaggtca gcctgacctg cctggtcaaa ggcttctatc ccagcgacat cgccgtggag     480

tgggagagca atgggcagcc ggagaacaac tacaagacca cgcctcccgt gctggactcc     540

gacggctcct tcttcctcta cagcaagctc accgtggaca agagcaggtg gcagcagggg     600

aacgtcttct catgctccgt gatgcacgag gctctgcaca accactacac gcagaagagc     660

ctctccctgt ctccgggtaa atga                                            684


<210>  19
<211>  227
<212>  PRT
<213>  Artificial Sequence

<220>
<223>  hIgG1 CH2/CH3

<400>  19

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Glu Glu Met Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  20
<211>  735

<212>  DNA
<213>  Artificial Sequence

<220>
<223>  DNA sequence of VH-linker-VL of CD3 scFv

<400>  20
caggtgcagc tgcagcagtc tggcgctgag ctggctagac ctggcgcctc cgtgaagatg      60

tcctgcaaga cctccggcta caccttcacc cggtacacca tgcactgggt caagcagagg     120

cctggacagg gcctggaatg gatcggctac atcaacccct cccggggcta caccaactac     180

aaccagaagt tcaaggacaa ggccaccctg acaaccgaca agtcctcctc caccgcctac     240

atgcagctgt cctccctgac ctccgaggac tccgccgtgt actactgcgc ccggtactac     300

gacgaccact actccctgga ctactggggc cagggcacca cactgacagt gtctagcgga     360

ggcggaggat ctggtggtgg cggatctggc ggcggtggaa gtggcggagg tggtagccag     420

atcgtgctga cccagtctcc cgccatcatg tctgctagcc ctggcgagaa agtgacaatg     480

acctgccggg cctcctcctc cgtgtcctac atgaactggt atcagcagaa gtccggcacc     540

tcccccaagc ggtggatcta cgacacctcc aaggtggcct ctggcgtgcc ctacagattc     600

tccggctctg gctctggcac ctcctacagc ctgaccatct ccagcatgga agccgaggat     660

gccgccacct actactgcca gcagtggtcc tccaaccccc tgacctttgg cgctggcacc     720

aagctggaac tgaag                                                      735

<210>  21
<211>  245
<212>  PRT
<213>  Artificial Sequence

<220>
<223>  amino acid sequence of VH-linker-VL of CD3 scFv

<400>  21

Gln Val Gln Leu Gln Gln Ser Gly Ala Glu Leu Ala Arg Pro Gly Ala
1               5                   10                  15

Ser Val Lys Met Ser Cys Lys Thr Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Ser Leu Asp Tyr Trp Gly Gln Gly
            100                 105                 110

Thr Thr Leu Thr Val Ser Ser Gly Gly Gly Gly Ser Gly Gly Gly Gly
        115                 120                 125

Ser Gly Gly Gly Gly Ser Gly Gly Gly Gly Ser Gln Ile Val Leu Thr
    130                 135                 140

Gln Ser Pro Ala Ile Met Ser Ala Ser Pro Gly Glu Lys Val Thr Met
145                 150                 155                 160

Thr Cys Arg Ala Ser Ser Ser Val Ser Tyr Met Asn Trp Tyr Gln Gln
                165                 170                 175

Lys Ser Gly Thr Ser Pro Lys Arg Trp Ile Tyr Asp Thr Ser Lys Val
            180                 185                 190

Ala Ser Gly Val Pro Tyr Arg Phe Ser Gly Ser Gly Ser Gly Thr Ser
        195                 200                 205

Tyr Ser Leu Thr Ile Ser Ser Met Glu Ala Glu Asp Ala Ala Thr Tyr
    210                 215                 220

Tyr Cys Gln Gln Trp Ser Ser Asn Pro Leu Thr Phe Gly Ala Gly Thr
225                 230                 235                 240

Lys Leu Glu Leu Lys
                245

<210>  22
<211>  32
<212>  PRT
<213>  Homo sapiens

<400>  22

Lys Gly Ser Gly Ala Glu Gly Leu Ile Pro Thr Val Leu Thr Leu Lys
1               5                   10                  15

Ala Ser Phe Lys Lys Leu Leu Phe Ile Gly Thr Ile Lys Val Gln Arg
            20                  25                  30

<210>  23
<211>  9
<212>  PRT

<213>  Artificial Sequence

<220>
<223>  natural antigen peptide derived from BORIS sf6

<400>  23

Leu Leu Phe Ile Gly Thr Ile Lys Val
1               5


<210>  24
<211>  8
<212>  PRT
<213>  Artificial Sequence

<220>
<223>  heavy chain CDR1 region of clone 11

<400>  24

Gly Phe Thr Phe Asp Lys Tyr Gly
1               5


<210>  25
<211>  8
<212>  PRT
<213>  Artificial Sequence

<220>
<223>  heavy chain CDR2 region of clone 11

<400>  25

Ile Thr Trp Asn Ser Gly Lys Val
1               5


<210>  26
<211>  25
<212>  PRT
<213>  Artificial Sequence

<220>
<223>  heavy chain CDR3 region of clone 11

<400>  26

Cys Ala Arg Asp Leu Gly Tyr Tyr Tyr Asp Ser Ser Gly Tyr Leu Lys
1               5                   10                  15


Pro Thr Gly Ser Gly Phe Asp Tyr Trp
            20                  25


<210>  27
<211>  9
<212>  PRT
<213>  Artificial Sequence

<220>

<223>  light chain CDR1 region of clone 11

<400>  27

Ser Ser Asn Ile Gly Ala Gly Tyr Asp
1               5


<210>  28
<211>  13
<212>  PRT
<213>  Artificial Sequence

<220>
<223>  light chain CDR3 region of clone 11

<400>  28


<210>  29
<211>  8
<212>  PRT
<213>  Artificial Sequence

<220>
<223>  heavy chain CDR1 region of clone 13

<400>  29

Gly Tyr Thr Phe Thr Gly Tyr Tyr
1               5


<210>  30
<211>  8
<212>  PRT
<213>  Artificial Sequence

<220>
<223>  heavy chain CDR2 region of clone 13

<400>  30

Ile Ser Thr Val Phe Asn Thr Pro
1               5


<210>  31
<211>  16
<212>  PRT
<213>  Artificial Sequence

<220>
<223>  heavy chain CDR3 region of clone 13

<400>  31

Cys Ala Arg Ser Tyr Ser Gly Ser Leu Gln Asp Ala Phe Asp Ile Trp
1               5                   10                  15

<210>  32
<211>  6
<212>  PRT
<213>  Artificial Sequence

<220>
<223>  light chain CDR1 region of clone 13

<400>  32

Asn Leu Lys Arg Lys Asn
1               5


<210>  33
<211>  13
<212>  PRT
<213>  Artificial Sequence

<220>
<223>  light chain CDR3 region of clone 13

<400>  33

Cys Gln Val Trp Asp Ile Asp Ser Glu Asp Tyr Val Phe
1               5                   10


<210>  34
<211>  8
<212>  PRT
<213>  Artificial Sequence

<220>
<223>  heavy chain CDR1 region of clone 19

<400>  34

Thr Tyr Ser Phe Ser Asn Tyr Trp
1               5


<210>  35
<211>  8
<212>  PRT
<213>  Artificial Sequence

<220>
<223>  heavy chain CDR2 region of clone 19

<400>  35

Ile Tyr Pro Gly Asp Pro Asp Thr
1               5


<210>  36
<211>  16
<212>  PRT
<213>  Artificial Sequence

<220>

<223>  heavy chain CDR3 region of clone 19

<400>  36

Cys Ala Arg Arg Trp Ala Ala Arg Pro Asp Asp Ala Phe Asp Ile Trp
1               5                   10                  15


<210>  37
<211>  6
<212>  PRT
<213>  Artificial Sequence

<220>
<223>  light chain CDR1 region of clone 19

<400>  37

Asn Ile Glu Ser Lys Ile
1               5


<210>  38
<211>  13
<212>  PRT
<213>  Artificial Sequence

<220>
<223>  light chain CDR3 region of clone 19

<400>  38


<210>  39
<211>  9
<212>  PRT
<213>  Artificial Sequence

<220>
<223>  HLA-A02 restricted peptide derived from HIV

<400>  39

Ser Leu Tyr Asn Thr Val Ala Thr Leu
1               5


<210>  40
<211>  9
<212>  PRT
<213>  Artificial Sequence

<220>
<223>  HLA-A02 restricted peptide derived from EBV

<400>  40

Tyr Leu Gln Gln Asn Trp Trp Thr Leu
1               5

<210>  41
<211>  9
<212>  PRT
<213>  Artificial Sequence

<220>
<223>  HLA-A02 restricted peptide derived from CMV

<400>  41

Asn Leu Val Pro Met Val Ala Thr Val
1               5